(19) Europäisches Patentamt
European Patent Office
Office européen des brevets

(11) **EP 4 512 831 A1**

(12) **EUROPEAN PATENT APPLICATION**
published in accordance with Art. 153(4) EPC

(43) Date of publication:
26.02.2025 Bulletin 2025/09

(51) International Patent Classification (IPC):
*C07K 16/46* (2006.01)     *C12N 15/13* (2006.01)
*A61K 39/395* (2006.01)     *A61P 35/00* (2006.01)

(21) Application number: 22938191.8

(22) Date of filing: 08.09.2022

(52) Cooperative Patent Classification (CPC):
A61K 39/395; A61K 47/68; A61P 35/00;
A61P 35/02; C07K 16/00; C07K 16/28;
C07K 16/46; C12N 5/10; C12N 15/85

(86) International application number:
PCT/CN2022/117681

(87) International publication number:
WO 2023/201966 (26.10.2023 Gazette 2023/43)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 20.04.2022 CN 202210414597

(71) Applicant: Regenecore Biotech Co., Ltd
Nanjing, Jiangsu 210000 (CN)

(72) Inventors:
• SU, Zhipeng
Nanjing, Jiangsu 210000 (CN)
• WANG, Yang
Nanjing, Jiangsu 210000 (CN)
• XIE, Wei
Nanjing, Jiangsu 210000 (CN)

(74) Representative: Murgitroyd & Company
165-169 Scotland Street
Glasgow G5 8PL (GB)

Remarks:
The complete document including Reference
Table(s) and the Sequence Listing(s) can be
downloaded from the EPO website

(54) **ANTIBODY COMPRISING IGG FC REGION VARIANT, AND USE THEREOF**

(57) Provided are an antibody comprising an IgG Fc region variant, and use thereof. The antibody comprising the IgG Fe region variant is a bispecific or multispecific antibody. The antibody comprises a first full-length antibody and a second full-length antibody. The first full-length antibody comprises a first antigen binding region and a modified first variant Fc region polypeptide. The second full-length antibody comprises a second antigen binding region and a modified second variant Fc region polypeptide. Further provided are a polynucleotide encoding the antibody, a host cell comprising the polynucleotide, and a method for preparing the antibody

Figure 2

**Description**

TECHNICAL FIELD

The present application relates to the field of biomedicine, and in particular to an antibody including an IgG Fc region variant and uses thereof.

BACKGROUND

There are five types of immunoglobulins in human: IgG, IgM, IgD, IgA and IgE. The most common isotype is IgG, which is composed of two identical heavy chain polypeptides and two identical light chain polypeptides. The two heavy chains are covalently linked to each other by disulfide bonds and each light chain is linked to a heavy chain by a disulfide bond. Each heavy chain contains four distinct domains that are generally referred to as variable domain (VH), constant heavy domain 1 (CH1), constant heavy domain 2 (CH2), and constant heavy domain 3 (CH3). The CH1 and CH2 domains are joined by a hinge region (an interdomain section) that provides the Ig with flexibility. The heavy chain constant domains, primarily CH2 and CH3, are involved in non-antigen binding functions. This region, generally known as the Fc region, has many important functions. For example, the Fc region binds complement, which may trigger phagocytosis or complement-dependent cytotoxicity (CDC). The Fc region also binds Fc receptors, which may trigger phagocytosis or antibody-dependent cellular cytotoxicity (ADCC). Antibody-dependent cell-mediated cytotoxicity (ADCC) refers to a form of cytotoxicity in which secreted IgG bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The Fc region also plays a role in helping to maintain the immunoglobulin in circulation and interacts with protein A, which is commonly used to purify immunoglobulin.

Efforts have been made in recent years to improve the immunogenic qualities and antigen binding characteristics of antibodies. For example, monoclonal, bispecific, and single-domain antibodies have been developed for immunotherapy. With the continuous development of biological medicines, therapeutic antibodies have become the main choice of drugs for patients with cancer, autoimmune disease, inflammation, and various other diseases. Monoclonal antibodies target a single target, and many patients cannot fully respond to a single therapy. Patients may develop resistance or no response to the drug. Cancer is different from other diseases. It is a multifactorial disease with multiple signaling pathways involved in the occurrence of the disease. Immunotherapy with a single target does not seem to be enough to destroy cancer cells. Therefore, bispecific antibodies, which have been in the hottest research and development in recent years, have gradually been recognized.

Bispecific antibodies (BsAb), composed of two physically connected antigen-binding portions, can interact with different antigenic epitopes on the same antigen or different antigens at the same time, exerting a synergistic effect that is more advantageous than monoclonal antibodies, and also mediating the occurrence of a variety of specific biological effects, for example, (1) bridging of immune cells and tumor cells, killing of tumor cells by recruiting and activating immune cells; (2) inhibition or stimulation of multiple signaling pathways to exert a coordinated effect; (3) mediation of the formation of protein complexes with the help of the bivalent structure of antibodies to exert biological effects, and so on. BsAb does not occur naturally, but is prepared via cell fusion or recombinant DNA technology. Due to its specificity and bifunctionality, it has become a research hotspot in the field of antibody engineering, and has wide application prospects in the fields such as tumor treatment and autoimmune diseases.

Acute leukemia is a malignant clonal disease of hematopoietic stem cells in which the leukemia cells stagnate at different stages of cell development due to their enhanced self-renewal, uncontrolled proliferation, differentiation disorders, and blocked apoptosis, leading to their proliferation and accumulation in large numbers and thus inhibition of normal hematopoiesis and infiltration to other organs and tissues. Acute myeloid leukemia (AML) is the most common and most fatal type of leukemia in adult acute leukemia patients. Although there has been a deeper understanding of AML in the past 40 years, the treatment of AML has not changed significantly compared to other blood tumors. At present, the cure rate for AML patients aged 60 years and under is 35% to 40%, while for AML patients aged over 60 years, only 5% to 15%. Decitabine and azacitidine are a class of small-molecule drugs for the treatment of AML that target epigenetic mechanisms. In addition to small molecule drugs, antibody drugs and cell immunotherapies are also important means for targeted therapy.

CD33, a 67-KD glycosylated transmembrane protein belonging to the Siglec family, is activated after cross-linking or ligand binding, and mediates inhibitory signals, regulates intracellular calcium mobilization, cell adhesion, leukemia cell apoptosis, myeloid cell maturation and cytokine production, etc. During differentiation, CD33 decreases on mature granulocytes but is retained on macrophages, monocytes and dendritic cells. CD33 is expressed limitedly in non-hematopoietic tissues, but highly in AML cells. In addition, it has been confirmed that CD33 is also expressed on leukemia progenitor cells, making this molecule a very potential therapeutic target for AML. Gentuzumabozogamicin is a derivative of human IgG4 CD33 monoclonal antibody (pH 67.6) coupled to calicheamicin with a Drug Antibody Ratio (DAR) of 2-3 and contains approximately 50% naked antibodies. It was approved by the FDA in September 2017 for use in adults with newly diagnosed CD33-positive acute myeloid leukemia (AML), adults with relapsed or refractory AML, and 2-year-old and pediatric patients with AML. IMGN779 (ImmunoGen, Inc., Waltham, Massachusetts) is composed of a CD33 monoclonal antibody coupled to indolinobenzodiazeprine pseudodimers (alkylating agents). A clinical study (NCT02674763) has been conducted, showing that 50 patients who has received IMGN779 reported no dose-limiting toxicity (DLT). Adverse events of treatment included febrile neutropenia, epistaxis, nausea, diarrhea, fatigue, abdominal pain, and hypokalemia. Overall, blast counts were reduced in 19 of 24 patients (79%) with measurable circulating blasts. These preliminary results showed that it was safe and effective. BI 836858 is a fully human IgG1 anti-CD33 antibody that enhances binding to FC$\gamma$RIIIa to increase its ADCC activity. Preliminary results from Phase 1b reported the results of 31 patients receiving BI836858, with five patients achieving complete response (CR) and another four achieving morphological leukemia-free status. AMG330 (Amgen) is a CD33/CD3 bispecific T cell engager (BiTE) antibody structure that directs cytotoxic T cells to CD33-expressing AML cells and kills the target cells. It has been shown to be effective in preclinical trials and is currently in Phase 1 clinical trials (NCT02520427). Johnson & Johnson's BiTE JNJ-67371244 is also in Phase 1 clinical trials (NCT03915379). 161533 TriKE (GT Biopharma) (CD16/IL-15/CD33) enhances natural killer cell function and induces anti-tumor responses targeting CD33 by targeting CD33-positive malignant cells and CD33 myeloid-derived suppressor cells (MDSC). Phase 1/2 clinical studies are currently underway for NCT03214666, GTB-3550 (CD16/IL-15/CD33) Tri-Specific Killer Engager (TriKE™) for High Risk Heme Malignancies. AML is a highly malignant blood tumor that highly expresses CD33. Currently, monospecific bispecific, tri-specific antibodies, and ADCs are all under development by some companies. Mylotarg has been approved by the FDA. Although their development, like all drug development, is fraught with difficulties, CD33 is still the most promising development target for AML.

CD123 is a glycoprotein containing 360 amino acids. It is also the $\alpha$ chain of the IL-3 receptor (interleukin-3 receptor, IL-3R), which, together with IL-3R$\beta$ (CD131), forms a high-affinity IL-3R. IL-3R, after specifically recognizing and binding to IL-3, can promote cell growth and proliferation, which is associated with the occurrence of tumors, allergic inflammation, and autoimmune diseases. Studies have confirmed that CD123 is an AML-related antigen, highly expressed in leukemia stem cells and low or not expressed on normal hematopoietic stem cells, and thus is a potential therapeutic target for AML. Leukemia cells in AML patients can spontaneously phosphorylate and activate STAT5, causing the proliferation and differentiation of the cells and the resistance to apoptosis. Therefore, high expression of CD123 is one of factors leading to poor-prognosis AML. CSL362 is a humanized anti-CD123 monoclonal antibody, which is a derivative of CSL360. It has an improved anti-leukemia effect by imparting a stronger affinity with CD16 on the surface of natural killer (NK) cells through antibody engineering technology, and enhancing the antibody-dependent cell-mediated cytotoxicity (ADCC) of NK cells. CSL362 have been found to significantly inhibit the proliferation of leukemia cells in AML-transplanted mice in animal experiments. Also, CSL-362 is also in Phase I clinical trials. In addition, there are some bispecific antibodies targeting CD123. MGD006 can target CD123 and CD3 at the same time. MGD006 has been confirmed in early clinical studies that it hsa anti-leukemia effect, and has been used in experimental treatment of refractory/relapsed AML. So far, monoclonal antibodies targeting CD123 have not caused obvious toxic and side effects in the treatment of AML, but no anti-CD123 antibodies have been officially used in clinical treatment. Their anti-leukemia effects in humans remain to be observed.

Single domain antibodies are promising molecules that can overcome some of the therapeutic limitations of conventional monoclonal antibodies. However, the generation of bispecific antibody molecules of this class is challenging and requires extensive protein engineering and development of manufacturing processes depending on the chosen antibody format. Conventional antibodies are composed of two identical heavy chains and two identical light chains. Co-transfection of a host cell with two different plasmids (one encoding the heavy chain and another one encoding the light chain) always results in antibodies with correctly assembled heavy and light chains. However, for IgG-like bispecific antibodies, transfection with single plasmids encoding two different heavy chains and two different light chains is required. This results in a significant fraction of post-production mispaired antibodies, since each heavy chain can homo- or hetero-dimerize and can in turn associate with two light chains. In total, there are 10 unique chain combinations that can be associated, only one of which is a BsAb with the correct assembly of the heavy and light chains. In addition to the stepwise purification of the desired bispecific molecules through affinity columns with immobilized antigens, it is difficult to remove undesired byproducts during downstream processing. Non-IgG-like bispecific antibodies do not include an FC portion and are primarily composed of a single polypeptide chain, which makes their upstream and downstream processing easier. Therefore, due to the lack of FcRn binding sites, these molecules cannot promote Fc-mediated effector functions and show faster clearance from the body. Based on the complexity of bispecific antibody molecules, it is very difficult to obtain a bispecific antibody with the right combination and make it have Fc-mediated functions.

SUMMARY

The FC$\gamma$RIIIa binding of an antibody or Fc region fusion polypeptide can be modified by changing the amino acid residues in the non-corresponding positions of each Fc region polypeptide, because the above-mentioned modifications together play a role in the FCγRIIIa binding. It is found in the present disclosure that it is conducive to the formation of a structurally stable heterodimer by forming a pairing structure with two variant Fc region polypeptides which are formed by preparing two human IgG Fc region polypeptides and modifying (mutating/substituting) amino acid residues of the two Fc region polypeptides respectively.

It is also found in the present disclosure that the two variant Fc region polypeptides can form a stable heterodimer when they are fused with the recognition regions (VHH or VH) of different antigens. The above heterodimer can be prepared into an antibody that presents an asymmetric structure with each chain capable of acting on different antigens. Therefore, the antibody with an asymmetric structure herein has not only a high structural stability, but also functional advantages over symmetrical bispecific antibodies or polyspecific antibodies. This specific antibody has excellent targeting function and ADCC effect. As reported herein, antibodies and Fc region fusion polypeptides with the combination of different antigen binding regions are effective in treatment of disease, such as cancer.

A first aspect of the present disclosure provides an IgG Fc region, including a first variant Fc region polypeptide and a second variant Fc region polypeptide, wherein a) the first variant Fc region polypeptide and the second variant Fc region polypeptide are both derived from human IgG Fc region polypeptides, b) the first variant Fc region polypeptide differs from the second variant Fc region polypeptide in amino acid(s); c) the first variant Fc region polypeptide differs from the human IgG Fc region polypeptide in at least one amino acid, and d) the second variant Fc region polypeptide differs from the human IgG Fc region polypeptide in at least one amino acid; and the first variant Fc region polypeptide is paired with the second variant Fc region polypeptide.

In the antibody including the IgG Fc region of the second aspect as described below, the modified first variant Fc region polypeptide is the first variant Fc region polypeptide. the modified second variant Fc region polypeptide is the second variant Fc region polypeptide.

The variant Fc region polypeptide (the first variant Fc region polypeptide or the second variant Fc region polypeptide) as mentioned above refers to an Fc region variant that, in certain embodiments, may be generated by introducing one or more other amino acid modifications into the Fc region of a human IgG antibody. The variant Fc region polypeptide may be derived from the Fc region sequence of a human IgG (e.g., the Fc region of human IgG1, IgG2, IgG3, or IgG4), wherein the Fc region sequence of the human IgG includes amino acid modifications (e.g., substitutions/mutations) at one or more amino acid positions.

In some embodiments, the first variant Fc region polypeptide differs from the second variant Fc region polypeptide in amino acid(s) located in a CH3 domain of the human IgG Fc region.

The human IgG Fc region includes a part of a hinge region, a CH2 region, and a CH3 region, wherein a) the first variant Fc region polypeptide differs from the human IgG Fc region polypeptide in amino acid(s) located in the CH3 domain of the human IgG Fc region; and

b) the second variant Fc region polypeptide differs from the human IgG Fc region polypeptide in amino acid(s) located in the CH3 domain of the human IgG Fc region.

In some embodiments, the human IgG Fc region polypeptide is derived from the Fc region polypeptide of human IgG1, IgG2, IgG3, or IgG4.

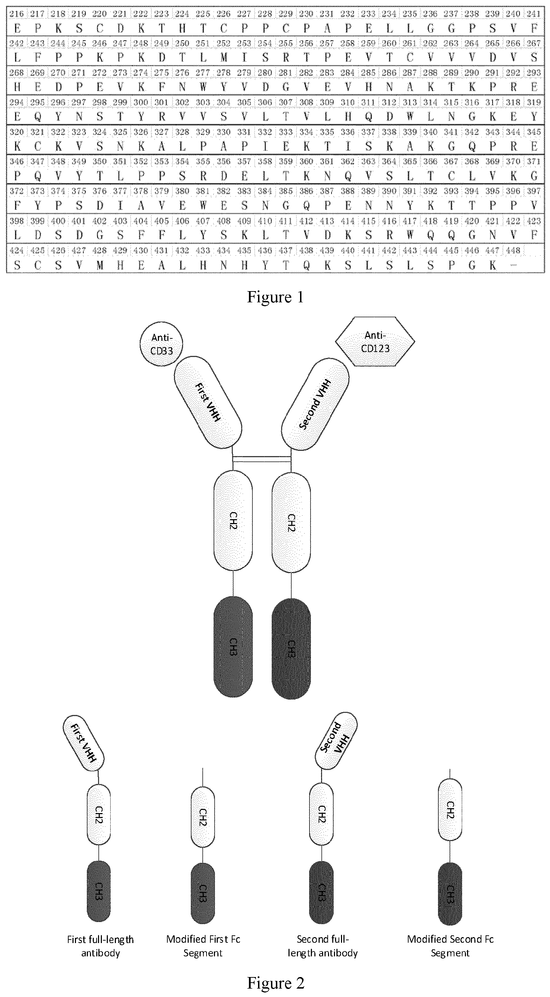

The Fc amino acid numbering follows the Kabat numbering. Taking the Fc region of wild-type human IgG as an example, "Kabat numbering" refers to the numbering system set forth by Kabat et al., U.S. Dept. of Health and Human Services, "Sequence of Proteins of Immunological Interest" (1983). The specific numbering is as shown in Figure 1, where amino acids 216-230 are the hinge domain, amino acids 231-340 are the domain of the second constant region CH2 of the heavy chain, and amino acids 341-447 are the domain of the third constant region CH3 of the heavy chain. In one embodiment, the wild-type human Fc region involved in the present disclosure is selected from IgG1, and the mutation is a mutation based on IgG1.

As used herein, an Fc region may possess "effector functions" that are responsible for activating or diminishing a biological activity (e.g. in a subject). Examples of effector functions include, but are not limited to: complement-dependent cytotoxicity (CDC); Fc receptor binding; antibody-dependent cell-mediated cytotoxicity (ADCC); phagocytosis; down regulation of cell surface receptors (e.g., B cell receptor; BCR), etc. Such effector functions may require the Fc region to be combined with a binding domain (e.g. an antibody variable domain) and can be assessed using various assays (e.g. Fc binding assay, ADCC assays, CDC assays, etc.).

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitutions providing functionally similar amino acids are well-known in the art.

Conservative substitution of amino acid is a substitution in which an amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Conservative modifications can be selected, for example, based on the similarity of polarity, charge, solubility, hydrophobicity, hydrophilicity, and/or the amphipathic nature of the residues involved.

A second aspect of the present disclosure provides an antibody including an IgG Fc region variant, wherein the antibody is a bispecific or multispecific antibody, and the antibody includes a first full-length antibody and a second full-length antibody, wherein the first full-length antibody includes a first antigen-binding region and a modified first variant Fc region polypeptide, and the second full-length antibody includes a second antigen-binding region and a modified second variant Fc region polypeptide, wherein the modified first variant Fc region polypeptide and the modified second variant Fc region polypeptide are each derived from human IgG Fc region polypeptides, and the first antigen-binding region and the second antigen-binding region are configured to recognize different antigens; wherein a carboxyl terminus of the first antigen-binding region is coupled to an amino terminus of the modified first variant Fc region polypeptide, and a carboxyl terminus of the second antigen-binding region is coupled to an amino terminus of the modified second variant Fc region polypeptide; the modified first variant Fc region polypeptide is paired with the modified second variant Fc region polypeptide in a manner selected from the group consisting of (as shown in Table 1):

1) the modified first variant Fc region polypeptide has an amino acid sequence as set forth in SEQ ID NO: 1 or having at least 80% homology to SEQ ID NO: 1, and the modified second variant Fc region polypeptide has an amino acid sequence as set forth in SEQ ID NO: 38 or having at least 80% homology to SEQ ID NO: 38; or

2) the modified first variant Fc region polypeptide has an amino acid sequence as set forth in SEQ ID NO: 2 or having at least 80% homology to SEQ ID NO: 2, and the modified second variant Fc region polypeptide has an amino acid sequence as set forth in SEQ ID NO: 39 or having at least 80% homology to SEQ ID NO: 39; or

3) the modified first variant Fc region polypeptide has an amino acid sequence as set forth in SEQ ID NO: 3 or having at least 80% homology to SEQ ID NO: 3, and the modified second variant Fc region polypeptide has an amino acid sequence as set forth in SEQ ID NO: 40 or having at least 80% homology to SEQ ID NO: 40; or

4) the modified first variant Fc region polypeptide has an amino acid sequence as set forth in SEQ ID NO: 4 or having at least 80% homology to SEQ ID NO: 4, and the modified second variant Fc region polypeptide has an amino acid sequence as set forth in SEQ ID NO: 41 or having at least 80% homology to SEQ ID NO: 41; or

5) the modified first variant Fc region polypeptide has an amino acid sequence as set forth in SEQ ID NO: 5 or having at least 80% homology to SEQ ID NO: 5, and the modified second variant Fc region polypeptide has an amino acid sequence as set forth in SEQ ID NO: 42 or having at least 80% homology to SEQ ID NO: 42; or

6) the modified first variant Fc region polypeptide has an amino acid sequence as set forth in SEQ ID NO: 6 or having at least 80% homology to SEQ ID NO: 6, and the modified second variant Fc region polypeptide has an amino acid sequence as set forth in SEQ ID NO: 43 or having at least 80% homology to SEQ ID NO: 43; or

7) the modified first variant Fc region polypeptide has an amino acid sequence as set forth in SEQ ID NO: 7 or having at least 80% homology to SEQ ID NO: 7, and the modified second variant Fc region polypeptide has an amino acid sequence as set forth in SEQ ID NO: 44 or having at least 80% homology to SEQ ID NO: 44; or

8) the modified first variant Fc region polypeptide has an amino acid sequence as set forth in SEQ ID NO: 8 or having at least 80% homology to SEQ ID NO: 8, and the modified second variant Fc region polypeptide has an amino acid sequence as set forth in SEQ ID NO: 45 or having at least 80% homology to SEQ ID NO: 45; or

9) the modified first variant Fc region polypeptide has an amino acid sequence as set forth in SEQ ID NO: 9 or having at least 80% homology to SEQ ID NO: 9, and the modified second variant Fc region polypeptide has an amino acid sequence as set forth in SEQ ID NO: 46 or having at least 80% homology to SEQ ID NO: 46; or

10) the modified first variant Fc region polypeptide has an amino acid sequence as set forth in SEQ ID NO: 10 or having at least 80% homology to SEQ ID NO: 10, and the modified second variant Fc region polypeptide has an amino acid sequence as set forth in SEQ ID NO: 47 or having at least 80% homology to SEQ ID NO: 47; or

11) the modified first variant Fc region polypeptide has an amino acid sequence as set forth in SEQ ID NO: 11 or having at least 80% homology to SEQ ID NO: 11, and the modified second variant Fc region polypeptide has an amino acid sequence as set forth in SEQ ID NO: 48 or having at least 80% homology to SEQ ID NO: 48; or

12) the modified first variant Fc region polypeptide has an amino acid sequence as set forth in SEQ ID NO: 12 or having at least 80% homology to SEQ ID NO: 12, and the modified second variant Fc region polypeptide has an amino acid sequence as set forth in SEQ ID NO: 49 or having at least 80% homology to SEQ ID NO: 49; or

13) the modified first variant Fc region polypeptide has an amino acid sequence as set forth in SEQ ID NO: 13 or having at least 80% homology to SEQ ID NO: 13, and the modified second variant Fc region polypeptide has an amino acid sequence as set forth in SEQ ID NO: 50 or having at least 80% homology to SEQ ID NO: 50; or

14) the modified first variant Fc region polypeptide has an amino acid sequence as set forth in SEQ ID NO: 14 or having at least 80% homology to SEQ ID NO: 14, and the modified second variant Fc region polypeptide has an amino acid sequence as set forth in SEQ ID NO: 51 or having at least 80% homology to SEQ ID NO: 51; or

15) the modified first variant Fc region polypeptide has an amino acid sequence as set forth in SEQ ID NO: 15 or having at least 80% homology to SEQ ID NO: 15, and the modified second variant Fc region polypeptide has an amino acid sequence as set forth in SEQ ID NO: 52 or having at least 80% homology to SEQ ID NO: 52; or

16) the modified first variant Fc region polypeptide has an amino acid sequence as set forth in SEQ ID NO: 16 or having at least 80% homology to SEQ ID NO: 16, and the modified second variant Fc region polypeptide has an amino acid sequence as set forth in SEQ ID NO: 53 or having at least 80% homology to SEQ ID NO: 53; or

17) the modified first variant Fc region polypeptide has an amino acid sequence as set forth in SEQ ID NO: 17 or having at least 80% homology to SEQ ID NO: 17, and the modified second variant Fc region polypeptide has an amino acid sequence as set forth in SEQ ID NO: 54 or having at least 80% homology to SEQ ID NO: 54; or

18) the modified first variant Fc region polypeptide has an amino acid sequence as set forth in SEQ ID NO: 18 or having at least 80% homology to SEQ ID NO: 18, and the modified second variant Fc region polypeptide has an amino acid sequence as set forth in SEQ ID NO: 55 or having at least 80% homology to SEQ ID NO: 55; or

19) the modified first variant Fc region polypeptide has an amino acid sequence as set forth in SEQ ID NO: 19 or having at least 80% homology to SEQ ID NO: 19, and the modified second variant Fc region polypeptide has an amino acid sequence as set forth in SEQ ID NO: 56 or having at least 80% homology to SEQ ID NO: 56; or

20) the modified first variant Fc region polypeptide has an amino acid sequence as set forth in SEQ ID NO: 20 or having at least 80% homology to SEQ ID NO: 20, and the modified second variant Fc region polypeptide has an amino acid sequence as set forth in SEQ ID NO: 57 or having at least 80% homology to SEQ ID NO: 57; or

21) the modified first variant Fc region polypeptide has an amino acid sequence as set forth in SEQ ID NO: 21 or having at least 80% homology to SEQ ID NO: 21, and the modified second variant Fc region polypeptide has an amino acid sequence as set forth in SEQ ID NO: 58 or having at least 80% homology to SEQ ID NO: 58; or

22) the modified first variant Fc region polypeptide has an amino acid sequence as set forth in SEQ ID NO: 22 or having at least 80% homology to SEQ ID NO: 22, and the modified second variant Fc region polypeptide has an amino acid sequence as set forth in SEQ ID NO: 59 or having at least 80% homology to SEQ ID NO: 59; or

23) the modified first variant Fc region polypeptide has an amino acid sequence as set forth in SEQ ID NO: 23 or having at least 80% homology to SEQ ID NO: 23, and the modified second variant Fc region polypeptide has an amino acid sequence as set forth in SEQ ID NO: 60 or having at least 80% homology to SEQ ID NO: 60; or

24) the modified first variant Fc region polypeptide has an amino acid sequence as set forth in SEQ ID NO: 24 or having at least 80% homology to SEQ ID NO: 24, and the modified second variant Fc region polypeptide has an amino acid sequence as set forth in SEQ ID NO: 61 or having at least 80% homology to SEQ ID NO: 61; or

25) the modified first variant Fc region polypeptide has an amino acid sequence as set forth in SEQ ID NO: 25 or having at least 80% homology to SEQ ID NO: 25, and the modified second variant Fc region polypeptide has an amino acid sequence as set forth in SEQ ID NO: 62 or having at least 80% homology to SEQ ID NO: 62; or

26) the modified first variant Fc region polypeptide has an amino acid sequence as set forth in SEQ ID NO: 26 or having at least 80% homology to SEQ ID NO: 26, and the modified second variant Fc region polypeptide has an amino acid sequence as set forth in SEQ ID NO: 63 or having at least 80% homology to SEQ ID NO: 63; or

27) the modified first variant Fc region polypeptide has an amino acid sequence as set forth in SEQ ID NO: 27 or having at least 80% homology to SEQ ID NO: 27, and the modified second variant Fc region polypeptide has an amino acid sequence as set forth in SEQ ID NO: 64 or having at least 80% homology to SEQ ID NO: 64; or

28) the modified first variant Fc region polypeptide has an amino acid sequence as set forth in SEQ ID NO: 28 or having at least 80% homology to SEQ ID NO: 28, and the modified second variant Fc region polypeptide has an amino acid sequence as set forth in SEQ ID NO: 65 or having at least 80% homology to SEQ ID NO: 65; or

29) the modified first variant Fc region polypeptide has an amino acid sequence as set forth in SEQ ID NO: 29 or having at least 80% homology to SEQ ID NO: 29, and the modified second variant Fc region polypeptide has an amino acid sequence as set forth in SEQ ID NO: 66 or having at least 80% homology to SEQ ID NO: 66; or

30) the modified first variant Fc region polypeptide has an amino acid sequence as set forth in SEQ ID NO: 30 or having at least 80% homology to SEQ ID NO: 30, and the modified second variant Fc region polypeptide has an amino acid sequence as set forth in SEQ ID NO: 67 or having at least 80% homology to SEQ ID NO: 67; or

31) the modified first variant Fc region polypeptide has an amino acid sequence as set forth in SEQ ID NO: 31 or having at least 80% homology to SEQ ID NO: 31, and the modified second variant Fc region polypeptide has an amino acid sequence as set forth in SEQ ID NO: 68 or having at least 80% homology to SEQ ID NO: 68; or

32) the modified first variant Fc region polypeptide has an amino acid sequence as set forth in SEQ ID NO: 32 or having at least 80% homology to SEQ ID NO: 32, and the modified second variant Fc region polypeptide has an amino acid sequence as set forth in SEQ ID NO: 69 or having at least 80% homology to SEQ ID NO: 69; or

33) the modified first variant Fc region polypeptide has an amino acid sequence as set forth in SEQ ID NO: 33 or having at least 80% homology to SEQ ID NO: 33, and the modified second variant Fc region polypeptide has an amino acid sequence as set forth in SEQ ID NO: 70 or having at least 80% homology to SEQ ID NO: 70; or

34) the modified first variant Fc region polypeptide has an amino acid sequence as set forth in SEQ ID NO: 34 or having at least 80% homology to SEQ ID NO: 34, and the modified second variant Fc region polypeptide has an amino acid sequence as set forth in SEQ ID NO: 71 or having at least 80% homology to SEQ ID NO: 71; or

35) the modified first variant Fc region polypeptide has an amino acid sequence as set forth in SEQ ID NO: 35 or having at least 80% homology to SEQ ID NO: 35, and the modified second variant Fc region polypeptide has an amino acid sequence as set forth in SEQ ID NO: 72 or having at least 80% homology to SEQ ID NO: 72; or

36) the modified first variant Fc region polypeptide has an amino acid sequence as set forth in SEQ ID NO: 36 or having at least 80% homology to SEQ ID NO: 36, and the modified second variant Fc region polypeptide has an amino acid sequence as set forth in SEQ ID NO: 73 or having at least 80% homology to SEQ ID NO: 73; or

37) the modified first variant Fc region polypeptide has an amino acid sequence as set forth in SEQ ID NO: 37 or having at least 80% homology to SEQ ID NO: 37, and the modified second variant Fc region polypeptide has an amino acid sequence as set forth in SEQ ID NO: 74 or having at least 80% homology to SEQ ID NO: 74.

Table 1 37 schemes for Fc pairing and mutation sites

| Strategies | Code | Mutation Sites | Strategies | Code | Mutation Sites |
|---|---|---|---|---|---|
| Hydrogen bond destruction + ion bond adjustment | BM#1 | T366W/D399V/F405T | Innovative ionic bond adjustment + base mutation | BM#21 | D399V/F405T/D356K |
| | | T366S/L368A/Y407V/K409W | | | K409W/K439E |
| | BM#2 | T366W/E357K/D399K | | BM#22 | D399V/F405T/E357K |
| | | T366S/L368A/Y407V/K370E/K409D | | | K409W/K370E |
| | BM#3 | T366V/D399V/T405T | | BM#23 | D399V/F405T/E357D |
| | | Y407A/K409W | | | K409W/K370Y |
| | BM#4 | T366V/E357K/D399K | | BM#24 | D356K/E357D/Y407A |
| | | Y407A/K370E/K409D | | | K439E/K370Y/K409V |
| | BM#5 | T366W/D356K | | BM#25 | D399K/E357D |
| | | T366S/L368A/Y407V/K439E | | | K409D/K370Y |
| | BM#6 | T366W/E357D | | BM#26 | D399V/F405T/D356K/E357K |
| | | T366S/L368A/Y407V/K370Y | | | K409W/K439E/K370E |
| | BM#7 | T366W/Q347R | | BM#27 | D399V/F405T/D356Ki E357D |
| | | T366S/L368A/Y407V/K360D | | | K409W/K439E/K370Y |
| | BM#8 | T366V/D356K | | BM#28 | D356K/D399K/E357D |
| | | Y407A/K439E | | | K439E/K409D/K370Y |
| | BM#9 | T366V/E357D | | BM#29 | Q347R/D356K/Y407A |
| | | Y407A/K370Y | | | K360D/K439EK409V |
| | BM#10 | T366V/Q347R | | BM#30 | Q347R/E357K/Y407A |
| | | Y407A/K360D | | | K360D/K3T0E/K409V |
| A/B chain exchange | BM#11 | T366W/K409W | | BM#31 | Q347R/D399K |
| | | T366S/L368A/Y407V/D399V/T405T | | | K360D/K409D |
| | BM#12 | T366W/K370F/K409D | | BM#32 | Q347R1E357D/Y407A |
| | | T366S/L368A/Y407V/E357K/D399K | | | K360D/K370Y/K409V |
| | BM#13 | T366V/K409W | Innovative strategies | BM#33 | T366D |
| | | Y407A/D399V/F405T | | | Y407K |

EP 4 512 831 A1

| Strategies | Code | Mutation Sites | Strategies | Code | Mutation Sites |
|---|---|---|---|---|---|
| | BM#14 | T366VJK170E/K409D | | BM#34 | D356V |
| | | Y407A/E357K/D399K | | | K439W |
| | BM#15 | T366W/K439E | | BM#35 | E357V |
| | | T366S/L368A/Y407V/D356K | | | K370W |
| | BM#16 | T366W/K370Y | | BM#36 | T394W |
| | | T366S/L368A/Y407V/E357D | | | T394S/V397A/FA05SA/Y40TV |
| | BM#17 | T366W/K360D | | BM#37 | Q347M |
| | | T366S/L368A/Y407V/Q347R | | | K360W |
| | BM#18 | T366V/K439E | | | / |
| | | Y407A/D356K | | | |
| | BM#19 | Y407A/K370Y | | | |
| | | T366V/E357D | | | |
| | BM#20 | T366V/K360D | | | |
| | | Y407A/Q347R | | | |

EP 4 512 831 A1

All of the above sequences can be replaced with sequences having "at least 80% homology" to the sequences or sequences with only one or a few amino acids substituted; preferably "at least 85% homology", more preferably "at least 90% homology", more preferably "at least 95% homology", and most preferably "at least 98% homology".

The above antibodies are antibody variants, and in certain embodiments, amino acid sequence variants of antibodies provided herein are contemplated. For example, bispecific antibodies or multi-specific antibodies with an asymmetric structure can be formed, and the antibodies have amino acid sequence variants exhibiting the desired (enhanced or reduced) ADCC effect. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., the desired ADCC effect of the first full-length antibody and the second full-length antibody for different antigens, respectively.

The sequence modifications in the above groups (1) to (37) involve 1) enhanced ADCC, or 2) reduced ADCC, or 3) retaining only the ADCC effect. Regardless of the need, what is required is that the formed antibody can exert the Fc-mediated function, the Fc-mediated function and the antigen targeting function do not affect each other, and the formed asymmetric antibody has a highly stable structure.

In some embodiments, the present disclosure provides an antibody including the IgG Fc region, wherein the antibody is a bispecific antibody. The first antigen binding region is a first VHH, and the second antigen binding region is a second VHH. The first VHH is fused to a modified first variant Fc region polypeptide, and the second VHH is fused to a modified second variant Fc region polypeptide.

As shown in Figure 2, an antibody provided in one embodiment of the present disclosure is a bispecific antibody that can target both CD33 and CD123 antigen molecules. The bispecific antibody is essentially a recombinant protein including a first full-length antibody and a second full-length antibody. Each full-length antibody is composed of a VHH segment (antigen binding domain), a part of a hinge region, a CH2 domain, and a CH3 domain in sequen. The part of the hinge region, the CH2 domain, and the CH3 domain constitute the Fc region. The VHH segment is connected to the CH2 domain via the hinge region.

The first VHH and the modified first variant Fc region polypeptide are located in the first full-length antibody, and the second VHH and the modified second variant Fc region polypeptide are located in the second full-length antibody. The first VHH can specifically bind to CD33 molecule, and the second VHH can specifically bind to CD123 molecule. The modified first variant Fc region polypeptide and the modified second variant Fc region polypeptide are derived from the Fc region of human IgG. The amino acids of two original human IgG Fc regions are each modified, forming different Fc region pairing groups (paired and linked by disulfide bonds, ionic bonds, hydrophobic forces, etc.) in (1) to (37). Taking group (1) as an example, the polypeptide having an amino acid sequence as set in SEQ ID NO: 1 or having at least 80% homology to SEQ ID NO: 1 as the modified first variant Fc region polypeptide is fused to the first VHH segment (that is, a carboxyl terminus of the first VHH segment is coupled to an amino terminus of the modified first variant Fc region polypeptide) to form the first full-length antibody, and the polypeptide having an amino acid sequence as set in SEQ ID NO: 38 or having at least 80% homology to SEQ ID NO: 38 as the modified second variant Fc region polypeptide is fused to the second VHH segment (that is, a carboxyl terminus of the second VHH segment is coupled to an amino terminus of the modified second variant Fc region polypeptide) to form the second full-length antibody. They form the final asymmetric bispecific antibody, with the Fc regions paired (37 pairing schemes in total).

In some embodiments, the first VHH and the second VHH as described above are both derived from single-domain antibodies (sdAbs), which are derived from naturally occurring single-domain antigen-binding molecules known as heavy chain antibody devoid of light chains (also referred to herein as "heavy chain-only antibodies"). The single-domain antibody is an antibody that naturally lacks light chains in the peripheral blood of camel. The antibody contains only one heavy chain variable region (VHH) and two conventional CH2 and CH3 regions, but are not as easily adhered to each other, or even aggregated into blocks, as artificially modified single chain antibody fragments (scFv). For clarity reasons, the variable domain derived from a heavy chain antibody naturally devoid of light chain is known herein as a VHH to distinguish it from the conventional VH region of four-chain immunoglobulins. Such a VHH molecule can be derived from antibodies raised in Camelidae species, for example in camel, llama, vigogne, dromedary, alpaca and guanaco as well as other species besides Camelidae that may produce heavy chain molecules naturally devoid of light chain. VHHs raised in the above-mentioned species are within the scope of the present disclosure. In other embodiments, VHH may be derived from condricthoids, and in other embodiments, VHH may be derived from sharks. A single domain antibody may also include only one heavy chain variable region (VHH) without CH2 and CH3 regions (e.g., SEQ ID NO. 149-150).

In some embodiments, the single domain antibody (sdAb) is recombinant, CDR-grafted, humanized, camelized, deimmunized, and/or generated in vitro (e.g., by phage display selection). In some embodiments, the sdAb is a human sdAb produced by a transgenic mouse or rat expressing a human heavy chain fragment.

In some embodiments, the VHH includes a naturally occurring sdAb or a VHH fragment thereof or a derivative thereof, such as a camel sdAb or a VHH fragment thereof, or a humanized sdAb or a VHH fragment thereof derived from a camel sdAb. In one embodiment, the sdAb is derived from a llama. In one embodiment, the sdAb is further engineered to remove sequences that are not typically present in human antibodies (such as CDR regions or CDR-FR junctions).

In some embodiments, the antibodies including IgG Fc region variants provided in the present disclosure are multispecific antibodies. After being modified by gene recombination technology, its first VHH segment and the second VHH segment can target multiple targets and can therefore occur as multispecific antibodies.

In some embodiments, the present disclosure provides a bispecific antibody including an IgG Fc region. The first antigen binding region is a first antibody, and the second antigen binding region is a second antibody. A heavy chain VH of the first antibody is fused to a modified first variant Fc region polypeptide, and a heavy chain VH of the second antibody is fused to a modified second variant Fc region polypeptide. The first antibody includes a heavy chain VH and a light chain VL, the second antibody includes a heavy chain VH and a light chain VL, and the first antibody and the second antibody target different antigens.

In some embodiments, the antibodies of the disclosure may target any combination of any antigens, the types of which include, but are not limited to, proteins, subunits, domains, motifs, and/or epitopes belonging to the following list of target antigens, which includes both soluble factors such as cytokines and membrane-bound factors, including transmembrane receptors: 17-IA, 4-1BB, 4Dc, 6-keto-PGFla, 8-iso-PGF2a, 8-oxo-dG, AI Adenosine Receptor, A33, ACE, ACE-2, Activin, Activin A, Activin AB, Activin B, Activin C, Activin RIA, Activin RIA ALK-2, Activin RIB ALK-4, Activin RIIA, Activin RIIB, ADAM, ADAM10, ADAM12, ADAM15, ADAM 17/TACE, ADAM8, ADAM9, ADAMTS, ADAMTS4, ADAMTS5, Addressins, aFGF, ALCAM, ALK, ALK-1, ALK-7, alpha- 1 -antitrypsin, alpha- V/beta- 1 antagonist, ANG, Ang, APAF-1, APE, APJ, APP, APRIL, AR, ARC, ART, Artemin, anti-Id, ASPARTIC, Atrial natriuretic factor, av/b3 integrin, Axl, b2M, B7-1, B7-2, B7-H, B-lymphocyte Stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2BMP-2a, BMP-3 Osteogenin, BMP-4BMP-2b, BMP-5, BMP-6Vgr-1, BMP-7(OP-1), BMP-8(BMP-8a, OP- 2), BMPR, BMPR-IA (ALK-3), BMPR-IB (ALK-6), BRK-2, RPK-1, BMPR-II (BRK-3), BMPs, b- NGF, BOK, Bombesin, Bone-derived neurotrophic factor, BPDE, BPDE-DNA, BTC, complement factor 3 (C3), C3a, C4, C5, C5a, C10, CA125, CAD-8, Calcitonin, cAMP, carcinoembryonic antigen (CEA), carcinoma-associated antigen, Cathepsin A, Cathepsin B, Cathepsin C/DPPI, Cathepsin D, Cathepsin E, Cathepsin H, Cathepsin L, Cathepsin O, Cathepsin S, Cathepsin V, Cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CDlla, CDllb, CDllc, CD13, CD14, CD15, CD16, CD18, CD19, CD20, CD21, CD22, CD23, CD25, CD27L, CD28, CD29, CD30, CD30L, CD32, CD33 (p67 proteins), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Clostridium botulinum toxin, Clostridiumperfringens toxin, CKb8-l, CLC, CMV, CMV UL, CNTF, CNTN-1, COX, C-Ret, CRG-2, CT-1, CTACK, CTGF, CTLA-4, CX3CL1, CX3CR1, CXCL, CXCLI, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor- associated antigen, DAN, DCC, DcR3, DC-SIGN, Decay accelerating factor, des(1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR(ErbB-1), EMA, EMMPRIN, ENA, endothelin receptor, Enkephalinase, eNOS, Eot, eotaxinl, EpCAM, Ephrin B2/EphB4, EPO, ERCC, E-selectin, ET-1, Factor 10a, Factor VII, Factor VIIIc, Factor IX, fibroblast activation protein (FAP), Fas, FcRI, FEN-1, Ferritin, FGF, FGF-19, FGF-2, FGF3, FGF-8, FGFR, FGFR-3, Fibrin, FL, FLIP, Flt-3, Flt-4, Follicle stimulating hormone, Fractalkine, FZD1, FZD2, FZD3, FZD4, FZD5, FZD6, FZD7, FZD8, FZD9, FZD10, G250, Gas 6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (Myostatin), GDF-9, GDF-15 (MIC-1), GDNF, GDNF, GFAP, GFRa-1, GFR-alphal, GFR-alpha2, GFR-alpha3, GITR, Glucagon, Glut 4, glycoprotein 10b/IIIa (GP10b/IIIa), GM-CSF, gpl30, gp72, GRO, Growth hormone releasing factor, Hapten (NP-cap or NIP-cap), HB-EGF, HCC, HCMV gB envelope glycoprotein, HCMV) gH envelope glycoprotein, HCMV UL, Hemopoietic growth factor (HGF), Hep B gpl20, heparanase, Her2, Her2/neu (ErbB-2), Her3 (ErbB-3), Her4 (ErbB-4), herpes simplex virus (HSV) gB glycoprotein, HSV gD glycoprotein, HGF A, high molecular weight melanoma-associated antigen (HMW-MAA), HIV gpl20, HIV IIIB gpl20 V3 loop, HLA, HLA-DR, HM1.24, HMFG PEM, HRG, Hrk, human cardiac myosin, human cytomegalovirus (HCMV), human growth hormone (HGH), HVEM, I-309, IAP, ICAM, ICAM-1, ICAM-3, ICE, ICOS, IFNg, Ig, IgA receptor, IgE, IGF, IGF binding proteins, IGF-1R, IGFBP, IGFI, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-23, interferon (INF)-alpha, INF -beta, INF-gamma, Inhibin, iNOS, Insulin A-chain, Insulin B-chain, Insulin- like growth factor 1, integrin alpha2, integrin alpha3, integrin alpha4, integrin alpha4/betal, integrin alpha4/beta7, integrin alpha5 (alphaV), integrin alpha5/betal, integrin alpha5/beta3, integrin alpha6, integrin betal, integrin beta2, interferon gamma, IP-10, I-TAC, JE, Kallikrein 2, Kallikrein 5, Kallikrein 6, Kallikrein 11, Kallikrein 12, Kallikrein 14, Kallikrein 15, Kallikrein LI, Kallikrein L2, Kallikrein L3, Kallikrein L4, KC, KDR, Keratinocyte Growth Factor (KGF), laminin 5, LAMP, LAP, LAP (TGF-1), Latent TGF-1, Latent TGF-1 bpl, LBP, LDGF, LECT2, Lefty, Lewis- Y antigen, Lewis- Y related antigen, LFA-1, LFA-3, Lfo, LIF, LIGHT, lipoproteins, LIX, LKN, Lptn, L-Selectin, LT-a, LT-b, LTB4, LTBP-1, Lung surfactant, Luteinizing hormone, Lymphotoxin Beta Receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Mucl), MUC18, Muellerian-inhibitin substance, Mug, MuSK, NAIP, NAP, NCAD, N-Cadherin, NCA 90, NCAM, NCAM, Neprilysin, Neurotrophin-3, -4, or -6, Neurturin, Neuronal growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, Parathyroid hormone, PARC, PARP, PBR, PBSF, PCAD, P-Cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, Proinsulin, Prorelaxin, Protein C, PS, PSA, PSCA, prostate specific membrane antigen (PSMA), PTEN, PTHrp, Ptk, PTN, R51, RANK, RANKL, RANTES, RANTES, Relaxin A- chain, Relaxin B-chain, renin, respiratory syncytial virus (RSV) F, RSV Fgp, Ret, Rheumatoid factors, RLIP76, RPA2, RSK, S100, SCF/KL, SDF-1, SERINE, Serum albumin, sFRP-3, Shh, SIGIRR, SK-1, SLAM, SLPI, SMAC, SMDF, SMOH, SOD, SPARC, Stat, STEAP, STEAPII, TACE, TACI, TAG-72 (tumor-associated glycoprotein-72), TARC, TCA-3, T-cell receptors (e.g., T-cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta1 , TGF-beta2, TGF-beta3, TGF-beta4, TGF-beta5, Thrombin, Thymus Ck-1 , Thyroid stimulating hormone, Tie, TIMP, TIQ, Tissue Factor, TMEFF2, Tmpo, TMPRSS2, TNF, TNF-alpha, TNF-alpha beta, TNF-beta2, TNFc, TNF-RI, TNF-RII, TNFRSF10A (TRAIL RI Apo-2, DR4), TNFRSF10B (TRAIL R2 DR5, KILLER, TRICK-2A, TRICK-B), TNFRSF10C (TRAIL R3 DcRI, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD 120a, p55-60), TNFRSF1B (TNF RII CD 120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APTI, CD95), TNFRSF6B (DcR3M68, TR6), TNFRSF7 (CD27), TNFRSF8 (CD30), TNFRSF9 (4-IBB CD 137, ILA), TNFRSF21 (DR6), TNFRSF22 (DcTRAIL R2TNFRH2), TNFRST23 (DcTRAILRITNFRH1), TNFRSF25 (DR3 Apo-3, LARD, TR-3, TRAMP, WSL-1), TNFSF10 (TRAIL Apo-2 Ligand, TL2), TNFSF11 (TRANCE/RANK Ligand ODF, OPG Ligand), TNFSF12 (TWEAK Apo-3 Ligand, DR3 Ligand), TNFSF13 (APRILTALL2), TNFSF13B (BAFF BLYS, TALL1, THANK, TNFSF20), TNFSF14 (LIGHT HVEM Ligand, LTg), TNFSF15 (TLIA/VEGI), TNFSF18 (GITR Ligand AITR Ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 Ligand gp34, TXGPI), TNFSF5 (CD40 Ligand CD154, gp39, HIGMI, IMD3, TRAP), TNFSF6 (Fas Ligand Apo-1 Ligand, APT1 Ligand), TNFSF7 (CD27 Ligand CD70), TNFSF8 (CD30 Ligand CD153), TNFSF9 (4- IBB Ligand CD 137 Ligand), TP-1, t-PA, Tpo, TRAIL, TRAIL R, TRAIL-R1, TRAIL- R2, TRANCE, transferring receptor, TRF, Trk, TROP-2, TSG, TSLP, tumor-associated antigen CA 125, tumor-associated antigen expressing Lewis Y related carbohydrate, TWEAK, TXB2, Ung, uPAR, uPAR-1, Urokinase, VCAM, VCAM-1, VECAD, VE-Cadherin, VE-cadherin-2, VEFGR-1 (flt-1), VEGF, VEGFR, VEGFR-3 (flt-4), VEGI, VIM, Viral antigens, VLA, VLA-1, VLA-4, VNR integrin, von Willebrands factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, and receptors for hormones and growth factors. The binding region for any one or several target antigens in the above target antigen list is located in the antigen binding region of the first full-length antibody in the bispecific antibody; and the antigen binding region for a target antigen different from at least one of the target antigens targeted by the first full-length antibody is located in the second full-length antibody of the bispecific antibody.

In some embodiments, the first antigen binding region targets CD33 molecule, and the second antigen binding region targets CD123 molecule.

In some embodiments, the first antigen binding region targets CD123 molecule, and the second antigen binding region targets CD33 molecule.

"CD33" referred to herein includes any recombinant or naturally occurring form of CD33, or a variant or homolog thereof that maintains CD33 activity (e.g., within at least 50%, 80%, 90%, 95%, 96%, 97%, 98%, 99% or 100% activity compared to CD33). In some aspects, the variant or homolog has at least 90%, 95%, 96%, 97%, 98%, 99% or 100% amino acid sequence identity in the entire sequence or a partial sequence (e.g., a 50, 100, 150 or 200 consecutive amino acid portion) compared to a naturally occurring CD33 protein.

"CD123" referred to herein includes any recombinant or naturally occurring form of CD123, or a variant or homolog thereof that maintains CD123 activity (e.g., within at least 50%, 80%, 90%, 95%, 96%, 97%, 98%, 99% or 100% activity compared to CD123). In some aspects, the variant or homolog has at least 90%, 95%, 96%, 97%, 98%, 99% or 100% amino acid sequence identity in the entire sequence or a partial sequence (e.g., a 50, 100, 150 or 200 consecutive amino acid portion) compared to a naturally occurring CD123 protein.

In some embodiments, the present disclosure provides a bispecific antibody including an IgG Fc region variant according to any of the above items, wherein the first antigen binding region (targeting CD33 molecules) has an amino acid sequence as set forth in SEQ ID NO: 149, and the second antigen binding region (targeting CD123 molecules) has an amino acid sequence as set forth in SEQ ID NO: 150. The modified Fc region in the bispecific antibody molecule is any one of the Fc region pairing groups (1) to (37). Taking pairing group (1) as an example, the polypeptide sequence having an amino acid sequence as set forth in SEQ ID NO: 149 is fused with the polypeptide having an amino acid sequence as set forth in SEQ ID NO: 1 to form a first full-length antibody, and the polypeptide sequence having an amino acid sequence as set forth in SEQ ID NO: 150 is fused with the polypeptide having an amino acid sequence as set forth in SEQ ID NO: 38 to form a second full-length antibody. The Fc region of the first full-length antibody is paired with the Fc region of the second full-length antibody. The bispecific antibodies finally formed is numbered as shown in Table 2.

In some embodiments, the present disclosure provides a bispecific antibody including an IgG Fc region variant according to any of the above items, wherein a nucleotide sequence encoding the first antigen binding region is as set forth in SEQ ID NO: 151, and a nucleotide sequence encoding the second antigen binding region is as set forth in SEQ ID NO: 152.

The term "encode" as it is applied to polynucleotides refers to a polynucleotide which "encodes" a polypeptide and which, in its native state or when manipulated by methods well known to those skilled in the art, can be transcribed and/or translated to produce the mRNA for the polypeptide and/or a fragment thereof. An antisense strand is a complement of such a nucleic acid, and an encoding sequence can be deduced therefrom.

A third aspect of the present disclosure provides a nucleotide molecule encoding the antibody including an IgG Fc region variant according to any of the above items.

In some embodiments, the present disclosure provides nucleotide sequences encoding the amino acid sequences of SEQ ID NO: 1-SEQ ID NO: 74, wherein the nucleotide sequences encoding the amino acid sequences of SEQ ID NO: 1 to SEQ ID NO: 74 are SEQ ID NO: 75 to SEQ ID NO: 148, respectively.

All of the sequences in the above embodiment can be replaced with sequences having "at least 80% homology" to the sequences or sequences with only one or a few amino acids substituted; preferably "at least 85% homology", more preferably "at least 90% homology", more preferably "at least 95% homology", and most preferably "at least 98% homology".

A fourth aspect of the present disclosure provides a vector including a nucleotide molecule encoding an antibody including an IgG Fc region variant according to any of the above items, or including a nucleotide molecule as set forth in SEQ ID NO: 75 to SEQ ID NO: 148, or including a nucleotide molecule as set forth in SEQ ID NO: 151 and SEQ ID NO: 152.

The "vector" means a construct that is capable of delivering, and, preferably, expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

A fifth aspect of the present disclosure provides a host cell, which is capable of expressing an antibody including an IgG Fc region variant according to any of the above items or includes a vector according to any of the above items.

In some embodiments, the host cell includes a prokaryotic cell, a eukaryotic cell, or a bacteriophage.

In some embodiments, the host cell is selected from the group consisting of Escherichia coli, yeast cells, mammalian cells, bacteriophages, or a combination thereof.

In some embodiments, the prokaryotic cell is selected from the group consisting of Escherichia coli, Bacillus subtilis, Lactobacillus, Streptomyces, Proteus mirabilis, or a combination thereof.

In some embodiments, the eukaryotic cell is selected from the group consisting of Pichia pastoris, Saccharomyces cerevisiae, Schizosaccharomyces pombe, Trichoderma, or a combination thereof.

In some embodiments, the eukaryotic cell is selected from the group consisting of cells of insect such as fall armyworm, cells of plant such as tobacco, BHK cells, CHO cells, COS cells, myeloma cells, or a combination thereof.

In some embodiments, the host cell is a suspension ExpiCHO-S cell.

In some embodiments, the host cells are suspension 293F cells.

A sixth aspect of the present disclosure also provides a method for producing the above-mentioned antibody, including: (a) culturing a host cell according to any of the above items and (b) recovering the antibody.

In some embodiments, an antibody obtained by the method for producing the above-mentioned antibody is provided.

The bispecific antibodies provided in the present application have certain advantages, such as good expression yield, stability, biological or pharmacological activity, and pharmacokinetic properties in mammalian cells (such as HEK293 cells and CHO cells). They can be used to treat diseases such as cancer.

The seventh aspect of the present disclosure provides an immunoconjugate including an antibody including an IgG Fc region variant according to any of the above items.

In some embodiments, the immunoconjugates provided herein further include a cytotoxin, a biologically active protein, or a radioactive isotope.

An eighth aspect of the present disclosure provides a pharmaceutical composition including an above-mentioned antibody including an IgG Fc region variant and a pharmaceutically acceptable carrier. Generally, the above-mentioned bispecific antibody including an IgG Fc region variant can be formulated in a non-toxic, inert and pharmaceutically acceptable aqueous carrier medium, wherein pH is generally determined according to the isoelectric point of the antibody (the pH of the aqueous carrier medium needs to deviate from the isoelectric point of the antibody and differ from the isoelectric point of the antibody by about 2). The formulated pharmaceutical composition can be administered via conventional routes, including, but not limited to, intravenous administration.

The pharmaceutical composition of the present disclosure can be directly bind CD33 and CD123 protein molecules, and can also have Fc-mediated functions, and the each of functions does not affect the other, so it can be used to effectively treat cancer-related diseases. In addition, the pharmaceutical composition of the present disclosure can also be used in combination with other cancer treatment agents.

The pharmaceutical composition of the present disclosure includes a safe and effective amount (such as 0.001 to 99 wt%, preferably 0.01 to 90 wt%, more preferably 0.1 to 80 wt%) of the aforementioned antibody including the IgG Fc region variant and the pharmaceutically acceptable carrier or excipient. Such carriers include, but are not limited to, saline, buffer, glucose, water, glycerol, ethanol, and combinations thereof. The pharmaceutical preparation should match the mode of administration. The pharmaceutical composition of the present disclosure can be prepared in the form of an injection, for example, by conventional methods using physiological saline or an aqueous solution containing glucose and other adjuvants. Pharmaceutical compositions such as injections and solutions are preferably manufactured under sterile conditions.

In some embodiments, the present disclosure provides an agent for treating cancer, which includes the above-mentioned antibody including an IgG Fc region variant as an active ingredient.

As used herein, the term "treatment" refers to both therapeutic treatment and prophylactic or preventative measures, wherein the object is to prevent or slow down (lessen) an undesired physiological change or disorder, such as the progression of cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

A ninth aspect of the present disclosure provides use of the above-mentioned antibody including an IgG Fc region variant or the above-mentioned pharmaceutical composition in the preparation of a medicament for treating a disease.

In an embodiment, the above-mentioned antibody including an IgG Fc region variant or the above-mentioned pharmaceutical composition is used for treating cancer.

In an embodiment, the above-mentioned antibody including an IgG Fc region variant or the above-mentioned pharmaceutical composition is used for treating blood cancer.

In an embodiment, the above-mentioned antibody including an IgG Fc region variant or the above-mentioned pharmaceutical composition is used for treating acute leukemia.

In an embodiment, the above-mentioned antibody including an IgG Fc region variant or the above-mentioned pharmaceutical composition is used for treating acute myeloid leukemia.

The term "cancer" refers to all types of cancer, neoplasm or malignant tumors found in mammals, including leukemias, lymphomas, melanomas, neuroendocrine tumors, carcinomas and sarcomas. Exemplary cancers that may be treated with an antibody, pharmaceutical composition provided herein include lymphoma (e.g., Mantel cell lymphoma, follicular lymphoma, diffuse large B-cell lymphoma, marginal zona lymphoma, Burkitt's lymphoma), sarcoma, bladder cancer, bone cancer, brain tumor, cervical cancer, colon cancer, esophageal cancer, gastric cancer, head and neck cancer, kidney cancer, myeloma, thyroid cancer, leukemia, prostate cancer, breast cancer, ovarian cancer, pancreatic cancer, liver cancer (e.g., hepatocellular carcinoma) , lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, leukemia (e.g., lymphoblastic leukemia, chronic lymphocytic leukemia, hairy cell leukemia), acute myeloid leukemia, lymphoma, B cell lymphoma, or multiple myeloma. Additional examples include, thyroid cancer, cancers of the endocrine system, brain cancer, breast cancer, cervix cancer, colon cancer, head & neck cancer, esophagus cancer, liver cancer, kidney cancer, lung cancer, non-small cell lung cancer, melanoma, mesothelioma, ovarian cancer , sarcoma, gastric cancer, uterus cancer or Medulloblastoma, Hodgkin's Disease, Non-Hodgkin's Lymphoma, multiple myeloma, neuroblastoma, glioma, glioblastoma multiforme, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, hepatocellular carcinoma, Paget's Disease of the Nipple, Phyllodes Tumors, Lobular Carcinoma, Ductal Carcinoma, cancer of the pancreatic stellate cells, cancer of the hepatic stellate cells, or prostate cancer.

As used herein, an "antibody" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

As used herein, "affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described in prior art and herein.

Beneficial effects

The IgG Fc region of the present disclosure is combined with different antigen binding regions to obtain an asymmetric bispecific antibody, which has a high degree of structural stability. Size exclusion chromatography (SEC) and differential scanning fluorescence quantification (DSF) are important parameters for evaluating protein stability. In the evaluation of the antibody of above embodiment, the SEC main peak ratio of the bispecific antibody obtained in the present disclosure can reach >90%, and the DSF melting temperature can reach >60°C, indicating that the above embodiments of bispecific antibodies can better overcome the heavy chain mismatch problem commonly existing in asymmetric bispecific antibodies, and the bispecific antibodies designed and formed in the above embodiments have good stability.

BRIEF DESCRIPTION OF THE DRAWINGS

In order to more clearly illustrate the technical solution of the present application, the drawings required for use in the embodiments are briefly introduced below. Obviously, for one of ordinary skills in the art, other drawings can be obtained based on these drawings without any creative work.

Figure 1 shows the numbering of amino acids of Fc based on the Kabat numbering system (the wild-type human Fc region is selected from IgG1).

Figure 2 is a schematic diagram of the structure of a bispecific antibody against CD33 and CD123 provided in the present application.

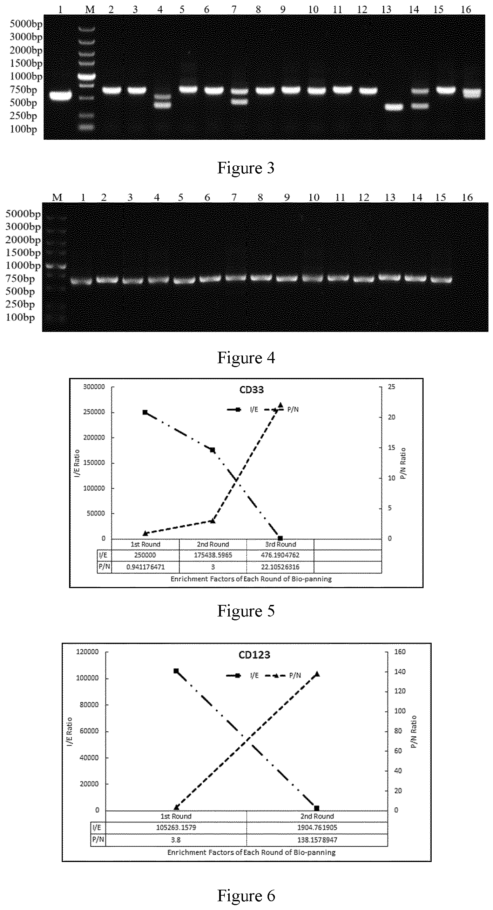

Figure 3 is an electrophoresis diagram of PCR products for determining the insertion rate of target fragments in the single domain antibody phage display library of CD33 protein. M represents DNA molecule marker, and numbers 1-16 represent PCR products of different colonies randomly selected from the constructed single domain antibody library against CD33 protein.

Figure 4 is an electrophoresis diagram of PCR products for determining the insertion rate of target fragments in the single domain antibody phage display library of CD123 protein. M represents DNA molecule marker. Numbers 1-16 represent PCR products of different colonies randomly selected from the constructed single domain antibody library against CD123 protein.

Figure 5 shows the specific results of bio-panning of the constructed single domain antibody library of CD33 protein. P/N = the number of monoclonal bacteria grown after TG1 bacteria infected with the phages eluted from the positive wells / the number of monoclonal bacteria grown after TG1 bacteria infected with the phages eluted from the negative wells in the bio-panning, this parameter will gradually increase after enrichment occurs. I/E = the total amount of phages added to the positive wells in each round of bio-panning / the total amount of phages eluted from the positive wells in each round of bio-panning, this parameter will gradually approach 1 after enrichment occurs.

Figure 6 shows the specific results of bio-panning of the constructed single domain antibody library of CD123 protein. P/N = the number of monoclonal bacteria grown after TG1 bacteria infected with the phages eluted from the positive wells / the number of monoclonal bacteria grown after TG1 bacteria infected with the phages eluted from the negative wells in the bio-panning, this parameter will gradually increase after enrichment occurs. I/E = the total amount of phages added to the positive wells in each round of bio-panning / the total amount of phages eluted from the positive wells in each round of bio-panning, this parameter will gradually approach 1 after enrichment occurs.

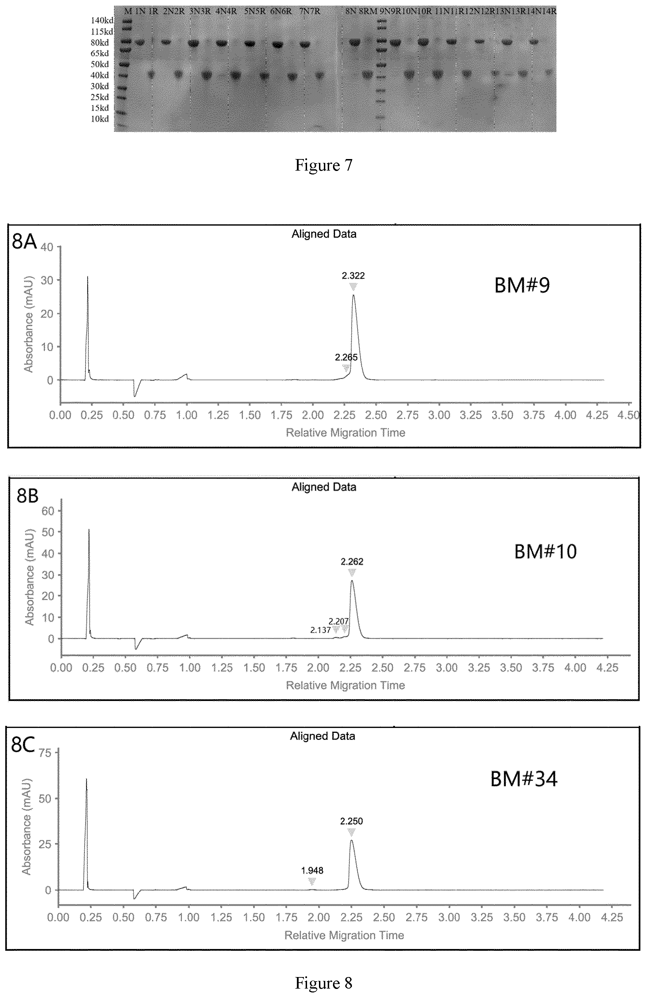

Figure 7 is an SDS-Page electrophoresis diagram of bispecific antibodies after Protein A purification. M represents protein Mark. Numbers 1-14 represent purified bispecific antibodies. N represents the antibody in non-reduced state, with band size of about 80Kd. R represents the antibody in reduced state, with band size of about 40Kd.

Figure 8 is a graph showing the results of non-reducing capillary electrophoresis detection of bispecific antibodies. The abscissa indicates the peak time, and the ordinate indicates the absorbance. The peak areas are calculated to show that the purities of the bispecific antibodies BM#9, BM#10, and BM#34 are 94.5%, 96.1%, and 98.4%, respectively.

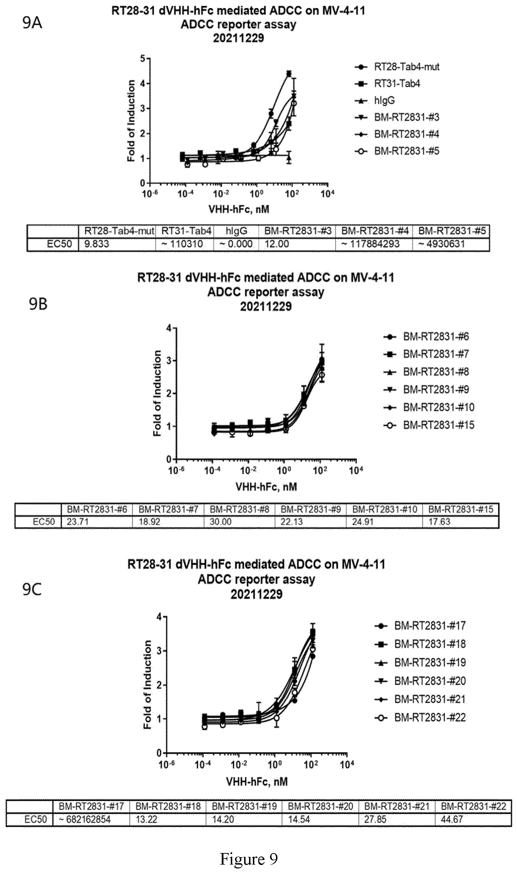

Figure 9 is a graph showing the ADCC function results of some bispecific antibodies determined by Reporter method. A smaller EC50 value indicates a stronger ADCC function of the antibody.

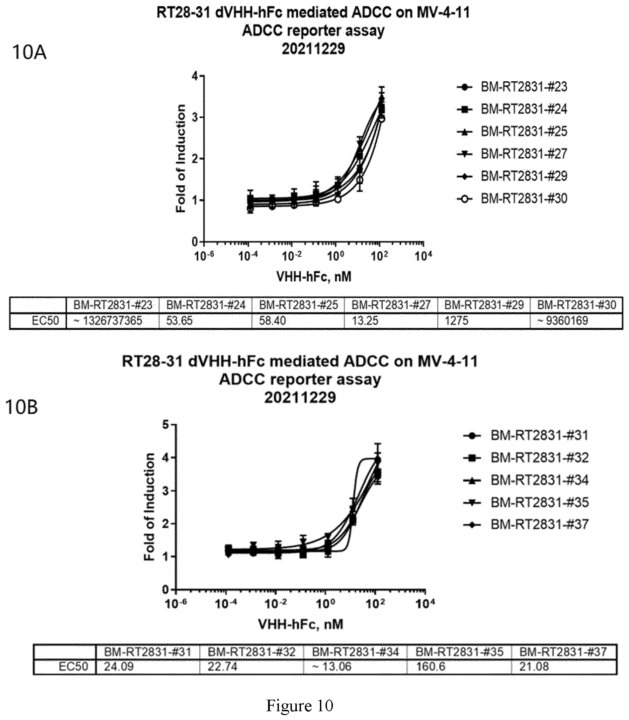

Figure 10 is a graph showing the ADCC function results of some other bispecific antibodies determined by Reporter method. A smaller EC50 value indicates a stronger ADCC function of the antibody.

DETAILED DESCRIPTION

The embodiments of the present application will be described in detail below in conjunction with the examples, but it is understood by those skilled in the art that the following examples are only for the purpose of illustrating the present application and should not be considered as limiting the scope of the present application. If specific conditions are not specified in the examples, they are carried out according to normal conditions or the conditions recommended by the manufacturer. If the manufacturer is not specified for the reagents or instruments used, they are all conventional products that can be obtained commercially.

Example 1: Construction of single domain antibody libraries targeting CD33 and CD123

Single domain antibody libraries targeting CD33 and CD123 were constructed as follows:

1. Recombinant proteins of CD33 and CD123 were prepared, respectively, as follows:

1.1 Preparation of CD33 recombinant protein: Using sequence-specific primers, the nucleotide sequence encoding amino acids 1 to 259 of CD33 protein was cloned into a vector pcDNA3.4 via restriction endonucleases XbaI and AgeI. The constructed vector was subjected to Sanger sequencing and compared with the original sequence. After confirmed, the recombinant plasmid was batch extracted for removal of endotoxins and transfected into suspension 293F to express the target protein (CD33 protein), followed by purification. The purified human recombinant CD33 protein has a purity as high as 90%, which meets the requirements for animal immunization.

1.2 Preparation of CD123 recombinant protein: Using sequence-specific primers, the nucleotide sequence encoding amino acids 19 to 300 of CD123 protein was cloned into a vector pcDNA3.4 via restriction endonucleases XbaI and AgeI. The constructed vector was subjected to Sanger sequencing and compared with the original sequence. After confirmed, the recombinant plasmid was batch extracted for removal of endotoxins and transfected into suspension 293F to express the target protein (CD123 protein), followed by purification. The purified human recombinant CD123 protein has a purity as high as 90%, which meets the requirements for animal immunization.

2. A single domain antibody library of CD33 was constructed as follows:

2.1. A Xinjiang Bactrian camel was immunized with 1 mg CD33 antigen emulsified and mixed with an equal volume of Freund's adjuvant once a week for a total of 7 consecutive immunizations. B cells were stimulated to express specific nano-antibodies in the process of immunization.

2.2. After immunization, 100 mL of camel peripheral blood lymphocytes were extracted and total RNA was extracted.

2.3. cDNA was synthesized and VHH was amplified using nested PCR.

2.4. 20 $\mu$g of pMECS phage display vector and 10 $\mu$g of VHH were digested using restriction endonucleases Pst I and Not I, and ligated (i.e., liagating the digested VHH and vector).

2.5. The ligation product was transformed into electroporation-competent TG1 cells to construct a CD33 protein phage display library. The library capacity was determined as about $2 \times 10^9$. Also, 16 colonies were randomly selected from the plate, and the correct insertion rate of the target fragment in the constructed library was determined by colony PCR. Figure 3 shows the result of colony PCR. M represents DNA molecule marker, and numbers 1-16 represent PCR products of different colonies randomly selected from the constructed single domain antibody library against CD33 protein, wherein 13 target bands have the same molecular weight as the theoretical value of 500bp-750bp. The results showed that the correct insertion rate reached 81.2% (1-3/16=81.2%).

2.6. A single domain antibody library of CD123 was constructed in the same way as constructing a CD33 library (basically the same as steps 2.1-2.5). Figure 4 shows an identification diagram of insertion rate for CD123 antibodies. M represents DNA molecule marker, and numbers 1-16 represent PCR products of different colonies randomly selected from the constructed single domain antibody library against CD123 protein, wherein 15 target bands have the same molecular weight as the theoretical value of 500bp-750bp. The results showed that the correct insertion rate reached 93.7% (1-1/16=93.7).

Example 2: Enrichment of single domain antibody libraries targeting CD33 and CD123

Single domain antibody library targeting CD33 was enriched as follows:

1. 200 $\mu$L of the recombinant TG1 cells were cultured in 2×TY medium. During this period, 40 $\mu$L of helper phage VCSM13 was added to infect TG1 cells. The cells were cultured overnight to allow the phages to be amplified. The next day, the phages were pelleted using PEG/NaCl and collected by centrifugation.

2. 100 $\mu$L of CD33 protein, which was diluted to 5 ng/$\mu$L in 0.1 M sterile $NaHCO_3$, was added to the positive wells, and 100 $\mu$L of sterile $NaHCO_3$ was added to the negative wells. They were incubated at 4°C overnight.

3. The next day, the overnight coated protein solution was pipetted and discarded. The plate was washed 10 times by adding 300 $\mu$L of 1‰ PBST to the wells and tapping the plate dry after each addition. Then 300 $\mu$L of 3% skim milk was added for blocking at room temperature for 2 hours.

4. After blocking, the milk was pipetted and discarded. The plate was washed 10 times with 300$\mu$L 1‰ PBST. Then 100$\mu$L amplified phage library (approximately $2×10^{11}$ phage particles) was added and incubated at room temperature for 1 hour.

5. After 1 hour of reaction, the plate was washed five times with 300 $\mu$L 1‰ PBST in order to wash away unbound phages.

6. The specifically bound phages were dissociated with trypsin at a final concentration of 0.25 mg/mL and used to infect E. coli TG1 cells in the logarithmic growth phase. The cells were then cultured at 37°C for 1 hour to generate phages, which was collected for the next round of screening. The same screening process was repeated twice for gradual enrichment. The enrichment effect of the CD33 single domain antibody library is shown in Figure 5. The P/N value increased from 0.9 in the first round to about 22 in the third round, and the I/E value decreased from 250,000 in the first round to about 476 in the third round, proving that the library has a very obvious enrichment for CD33 single domain antibodies.

7. The enrichment steps for CD123 single domain antibody library were the same as CD123, with the effect as shown in Figure 6. The P/N value increased from 3.8 in the first round to about 138 in the second round, and the I/E value decreased from 105263 in the first round to about 1904 in the second round, proving that the library has a very obvious enrichment for CD33 single domain antibodies. In Figures 5 and 6, P/N = the number of monoclonal bacteria grown after TG1 bacteria infected with the phages eluted from the positive wells / the number of monoclonal bacteria grown after TG1 bacteria infected with the phages eluted from the positive wells in the bio-panning, this parameter will gradually increase after enrichment occurs. I/E = the total amount of phages added to the positive wells in each round of bio-panning / the total amount of phages eluted from the positive wells in each round of bio-panning, this parameter will gradually approach 1 after enrichment occurs.

Example 3: Screening of specific positive clones using phage enzyme-linked immunosorbent assay (ELISA)

1. According to the above-mentioned single-domain antibody screening method, CD33 or CD123 was screened for multiple rounds. After the screening, the phage enrichment factor for CD33 or CD123 reached more than 10 (about 10,000). 400 single colonies were selected from the positive clones obtained by screening and inoculated in TB medium containing 100 $\mu$g/mL ampicillin in 96 deep-well plates, with blank control also set in the plates. After culturing at 37°C to the logarithmic phase, IPTG was added to a final concentration of 1 mM and the plates were incubated at 28 °C overnight. The crude antibody was obtained by osmotic bursting.

2. CD33 protein or CD123 protein was diluted into 100 mM $NaHCO_3$ at pH 8.3. An ELISA plate was coated with 100 $\mu$g CD33 protein at 4 °C overnight. The next day, 100 $\mu$g BSA-Biotin protein was added to the ELISA plate.

3. 100 $\mu$L of crude antibody extract obtained in the above step was transferred to the ELISA plate into which the antigen was added, and incubated at room temperature for 1 h.

4. The unbound antibodies were washed away with PBST. 100 $\mu$L of mouse anti-HA tag antibody (purchased from Thermo Fisher) at 1:2000 dilution was added, and incubated at room temperature for 1 h.

5. The unbound antibodies were washed away with PBST. 100 $\mu$L of anti-rabbit HRP conjugate (horseradish peroxidase-labeled goat anti-rabbit antibody, purchased from Thermo Fisher) in 1:20000 dilution was added, and incubated at room temperature for 1 h.

6. The unbound antibodies were washed away with PBST. A horseradish peroxidase color development solution was added. After reaction at 37° C for 15 min, a stopping solution was added, and the absorbance OD450 was measured at a wavelength of 450 nm in a microplate reader.

7. When the optical density (OD) of sample wells was more than 5 times that of control wells, the wells were determined as positive clone wells.

8. The bacteria in the positive clone wells were transferred into an LB medium containing 100 μg/L ampicillin on a shaker for plasmid extraction and sequencing.

The gene sequence of each clone was analyzed according to the sequence alignment software Vector NTI. The strains with the same CDR1, CDR2, and CDR3 sequences are regarded as the same clone, and the strains with different sequences are regarded as different clones, to finally obtain single-domain antibodies against CD33 or CD123. The antibody has an amino acid sequence of FR1-CDR1-FR2-CDR2-FR3-CDR3-FR4, to form the entire VHH. The obtained single-domain antibody recombinant plasmid can be expressed in a prokaryotic system to finally obtain a single-domain antibody protein.

The amino acid sequence of the screened anti-CD33 single domain antibody is as set forth in SEQ ID NO: 149; and the amino acid sequence of the screened anti- CD123 single domain antibody is as set forth in SEQ ID NO: 150.

Example 4: Construction of Eukaryotic Expression Vectors of Fc Fusion antibodies

The antibodies in Example 3 were fused to FC to prepare FC fusion antibodies. The eukaryotic vectors were constructed as follows:

1. The antibodies selected in Example 3 were sequenced by Sanger sequencing to obtain their nucleotide sequences (the nucleotide sequence of the anti-CD33 single domain antibody is SEQ ID NO: 151; and the nucleotide sequence of the anti- CD123 single domain antibody is SEQ ID NO: 152).

2. The above nucleotide sequences were each synthesized with the nucleotide sequence of the Fc variant into a vector pCDNA3.4 by sequence synthesis, thereby obtaining a plasmid containing the nucleotide sequence encoding the first full-length antibody and a plasmid containing the nucleotide sequence encoding the second full-length antibody, respectively.

The plasmid containing the nucleotide sequence encoding the first full-length antibody can be obtained according to a method including: (1) inserting the nucleotide sequence of the anti-CD33 single domain antibody as set forth in SEQ ID NO: 151 into the vector pCDNA3.4, and then inserting the nucleotide sequence of the corresponding Fc variant (i.e., modified first variant Fc region polypeptide); or (2) inserting at one time the nucleotide sequence of the anti-CD33 single domain antibody and the nucleotide sequence of the FC variant (i.e., the modified first variant Fc region polypeptide) into the vector pCDNA3.4. The plasmid containing the nucleotide sequence encoding the second full-length antibody can be obtained according to a method including: (1) inserting the nucleotide sequence of the anti-CD123 single domain antibody as set forth in SEQ ID NO: 152 into the vector pCDNA3.4, and then inserting the nucleotide sequence of the corresponding Fc variant (i.e., modified second variant Fc region polypeptide); or (2) inserting at one time the nucleotide sequence of the anti-CD123 single domain antibody and the nucleotide sequence of the FC variant (i.e., the modified second variant Fc region polypeptide) into the vector pCDNA3.4. For example, in the above nucleotide sequences and the nucleotide sequence of the Fc variant, the nucleotide sequence of the anti-CD33 single domain antibody is located at the 5' end of the nucleotide sequence of the first full-length antibody, the nucleotide sequence of the modified first variant Fc region is located at the 3' end of the nucleotide sequence of the first full-length antibody, and the nucleotide sequence of the first full-length antibody is directly synthesized as a whole; and the nucleotide sequence of the anti-CD123 single domain antibody is located at the 5' end of the nucleotide sequence of the second full-length antibody, the nucleotide sequence of the modified second variant Fc region is located at the 3' end of the nucleotide sequence of the second full-length antibody, and the nucleotide sequence of the second full-length antibody is directly synthesized as a whole.

SEQ ID NO:75-148 and SEQ ID NO:151-152 were all recited in an order of 5' to 3'.

3. The constructed recombinant eukaryotic expression vector was transformed into DH5α Escherichia coli, which were cultured for maxiprep extraction of CD33 or CD123 plasmid, respectively, thereby obtaining the plasmid containing the nucleotide sequence encoding the first full-length antibody and the plasmid containing the nucleotide sequence encoding the second full-length antibody, respectively, with the endotoxins removed.

4. The maxiprep extracted plasmid was sequenced for identification.

5. The confirmed recombinant vector was stored for subsequent eukaryotic cell transfection and expression. The bispecific antibody against CD33 and CD123 was obtained following the transfection and expression process.

pCDNA3.4 (vector data link: https://assets.thermofisher.com/TFS-Assets/LSG/manuals/pcdna3_4_topo_ta_cloning_kit_man.pdf). In addition, the vectors that can be used include but are not limited to pCDNA3.4, and can also be other vectors.

The bispecific antibodies against CD33 and CD123 produced in the above steps are shown in Figure 2. Table 2 shows the serial numbers of the bispecific antibodies.

Table 2 Serial numbers of the bispecific antibodies

| Serial number | First full-length antibody | | Second full-length antibody | |
|---|---|---|---|---|
| | Amino acid sequence of the first VHH | Amino acid sequence of the modified first variant Fc region | Amino acid sequence of the second VHH | Amino acid sequence of the modified second variant Fc region |
| BM#1 | SEQ ID NO: 149 | SEQ ID NO: 1 | SEQ ID NO: 150 | SEQ ID NO: 38 |
| BM#2 | SEQ ID NO: 149 | SEQ ID NO: 2 | SEQ ID NO: 150 | SEQ ID NO: 39 |
| BM#3 | SEQ ID NO: 149 | SEQ ID NO: 3 | SEQ ID NO: 150 | SEQ ID NO: 40 |
| BM#4 | SEQ ID NO: 149 | SEQ ID NO: 4 | SEQ ID NO: 150 | SEQ ID NO: 41 |
| BM#5 | SEQ ID NO: 149 | SEQ ID NO: 5 | SEQ ID NO: 150 | SEQ ID NO: 42 |
| BM#6 | SEQ ID NO: 149 | SEQ ID NO: 6 | SEQ ID NO: 150 | SEQ ID NO: 43 |
| BM#7 | SEQ ID NO: 149 | SEQ ID NO: 7 | SEQ ID NO: 150 | SEQ ID NO: 44 |
| BM#8 | SEQ ID NO: 149 | SEQ ID NO: 8 | SEQ ID NO: 150 | SEQ ID NO: 45 |
| BM#9 | SEQ ID NO: 149 | SEQ ID NO: 9 | SEQ ID NO: 150 | SEQ ID NO: 46 |
| BM#10 | SEQ ID NO: 149 | SEQ ID NO: 10 | SEQ ID NO: 150 | SEQ ID NO: 47 |
| BM#11 | SEQ ID NO: 149 | SEQ ID NO: 11 | SEQ ID NO: 150 | SEQ ID NO: 48 |
| BM#12 | SEQ ID NO: 149 | SEQ ID NO: 12 | SEQ ID NO: 150 | SEQ ID NO: 49 |
| BM#13 | SEQ ID NO: 149 | SEQ ID NO: 13 | SEQ ID NO: 150 | SEQ ID NO: 50 |
| BM#14 | SEQ ID NO: 149 | SEQ ID NO: 14 | SEQ ID NO: 150 | SEQ ID NO: 51 |
| BM#15 | SEQ ID NO: 149 | SEQ ID NO: 15 | SEQ ID NO: 150 | SEQ ID NO: 52 |
| BM#16 | SEQ ID NO: 149 | SEQ ID NO: 16 | SEQ ID NO: 150 | SEQ ID NO: 53 |
| BM#17 | SEQ ID NO: 149 | SEQ ID NO: 17 | SEQ ID NO: 150 | SEQ ID NO: 54 |
| BM#18 | SEQ ID NO: 149 | SEQ ID NO: 18 | SEQ ID NO: 150 | SEQ ID NO: 55 |
| BM#19 | SEQ ID NO: 149 | SEQ ID NO: 19 | SEQ ID NO: 150 | SEQ ID NO: 56 |
| BM#20 | SEQ ID NO: 149 | SEQ ID NO: 20 | SEQ ID NO: 150 | SEQ ID NO: 57 |
| BM#21 | SEQ ID NO: 149 | SEQ ID NO: 21 | SEQ ID NO: 150 | SEQ ID NO: 58 |
| BM#22 | SEQ ID NO: 149 | SEQ ID NO: 22 | SEQ ID NO: 150 | SEQ ID NO: 59 |
| BM#23 | SEQ ID NO: 149 | SEQ ID NO: 23 | SEQ ID NO: 150 | SEQ ID NO: 60 |
| BM#24 | SEQ ID NO: 149 | SEQ ID NO: 24 | SEQ ID NO: 150 | SEQ ID NO: 61 |
| BM#25 | SEQ ID NO: 149 | SEQ ID NO: 25 | SEQ ID NO: 150 | SEQ ID NO: 62 |
| BM#26 | SEQ ID NO: 149 | SEQ ID NO: 26 | SEQ ID NO: 150 | SEQ ID NO: 63 |
| BM#27 | SEQ ID NO: 149 | SEQ ID NO: 27 | SEQ ID NO: 150 | SEQ ID NO: 64 |
| BM#28 | SEQ ID NO: 149 | SEQ ID NO: 28 | SEQ ID NO: 150 | SEQ ID NO: 65 |
| BM#29 | SEQ ID NO: 149 | SEQ ID NO: 29 | SEQ ID NO: 150 | SEQ ID NO: 66 |
| BM#30 | SEQ ID NO: 149 | SEQ ID NO: 30 | SEQ ID NO: 150 | SEQ ID NO: 67 |
| BM#31 | SEQ ID NO: 149 | SEQ ID NO: 31 | SEQ ID NO: 150 | SEQ ID NO: 68 |
| BM#32 | SEQ ID NO: 149 | SEQ ID NO: 32 | SEQ ID NO: 150 | SEQ ID NO: 69 |

(continued)

| Serial number | First full-length antibody | | Second full-length antibody | |
|---|---|---|---|---|
| | Amino acid sequence of the first VHH | Amino acid sequence of the modified first variant Fc region | Amino acid sequence of the second VHH | Amino acid sequence of the modified second variant Fc region |
| BM#33 | SEQ ID NO: 149 | SEQ ID NO: 33 | SEQ ID NO: 150 | SEQ ID NO: 70 |
| BM#34 | SEQ ID NO: 149 | SEQ ID NO: 34 | SEQ ID NO: 150 | SEQ ID NO: 71 |
| BM#35 | SEQ ID NO: 149 | SEQ ID NO: 35 | SEQ ID NO: 150 | SEQ ID NO: 72 |
| BM#36 | SEQ ID NO: 149 | SEQ ID NO: 36 | SEQ ID NO: 150 | SEQ ID NO: 73 |
| BM#37 | SEQ ID NO: 149 | SEQ ID NO: 37 | SEQ ID NO: 150 | SEQ ID NO: 74 |

Example 5: Eukaryotic expression of bispecific antibodies

The eukaryotic expression of the bispecific antibodies BM#1-BM#37 in Example 4 was performed as follows:

1. 3 days before transfection, the 293F cells at $2.5 \times 10^5$/mL were passaged and expanded. The cells in a required volume by calculation were transferred into a 500 mL shake flask containing 120 mL (final volume) of fresh pre-warmed OPM-293 CD05 Medium. The cells were incubated to a concentration of about $2 \times 10^6$ to $3 \times 10^6$ living cells/mL.
2. On the day of transfection, the cell density and living cell percentage were determined. Before the transfection, the cell density should reach about $2 \times 10^6$ to $3 \times 10^6$ living cells/mL.
3. The cells were diluted to $1 \times 10^6$ living cells/mL with fresh pre-warmed OPM-293 CD05 Medium. The cells in a required volume by calculation were transferred into a 500 mL shake flask containing 100 mL (final volume) of medium.
4. PEI (1 mg/mL) reagent was diluted in 4 ml Opti-MEM medium, and mixed by shaking or pipetting; CD33 plasmid and CD123 plasmid (i.e., the plasmid containing the nucleotide sequence encoding the first full-length antibody and the plasmid containing the nucleotide sequence encoding the second full-length antibody) were mixed at a ratio of 1: 1, and diluted in 4 mL Opt-MEM medium, mixed by shaking or pipetting, filtered through a 0.22 $\mu$m filter, and incubated at room temperature for 5 minutes.
5. The diluted PEI reagent was added to the diluted plasmid and mixed by inversion. The PEI/plasmid DNA mixture was incubated at room temperature for 15 to 20 min, and then gently added into a prepared cell suspension during which the shake flask was gently shaken.
6. The cells were cultured under shaking at 120 rpm in 5% $CO_2$ at 37°C.
7. 5 mL of OPM-CHO PFF05 feed was added at 24h and 72h after transfection.
8. The supernatant, which contains the bispecific antibody of the present disclosure, was collected on day 7th after transfection (cell viability was less than 70%). The plasmid containing the nucleotide sequence encoding the first full-length antibody and the plasmid containing the nucleotide sequence encoding the second full-length antibody expressed to obtain the first full-length antibody and the second full-length antibody, respectively. In this process, the Fc region of the first full-length antibody and the Fc region of the second full-length antibody are naturally paired to form a bispecific antibody.

Example 6: Purification of bispecific antibodies

The bispecific antibody was purified as follows:

1. The expression supernatant of the protein obtained in Example 5 was filtered through a 0.45 $\mu$m disposable filter head to remove insoluble impurities.
2. The above filtrate was purified by affinity chromatography using a protein purifier, with an agarose filler conjugated to Protein A based on the binding of human Fc to Protein A.
3. The filtrate was allowed to pass through a Protein A prepacked column at a flow rate of 1 mL/min. In this step, the target protein in the filtrate binds to the filler.
4. The impurity proteins binding to the column were washed away with low-salt and high-salt buffers.
5. The target protein binding to the column was eluted with a low-pH buffer.
6. The eluted solution was quickly added in a Tris-HCl solution at pH 9.0 for neutralization.

7. The neutralized protein solution was dialyzed and then subjected to SDS-PAGE electrophoresis. As shown in Figure 7, Numbers 1-14 represent antibodies BM#1 to BM#14 after Protein A purification. N represents the sample in non-reduced state, with band size of about 80 Kd. R represents the sample in reduced state, with band size of about 40 Kd. The samples with the antibody concentration>0.5 mg/mL were preferably subjected to HPLC-SEC (size exclusion chromatography) to test the antibody purity. The peak area calculation showed that the antibody purity was above 90%. Also, DSF (differential scanning fluorescence) was used to test the thermal stability of the antibody, showing that the Tm value was>60°C, indicating good thermal stability. The results are shown in Table 6.

When testing the purity of antibodies, a high performance liquid chromatograph (Thermo Fisher U3000) was used. HPLC-SEC (size exclusion chromatography) detection method was performed as follows:

a. Solution preparation

(1) Mobile phase A: 3.3 g of disodium hydrogen phosphate, 3.2 g of sodium dihydrogen phosphate, and 17.52 g of sodium chloride were accurately weighed and added to about 800 ml of ultrapure water under stirring for dissolution, with pH adjusted to 6.8. Water was added to make up to 1 L. After filtered through a 0.22 µm water-based microporous filter membrane, the solution was stored at room temperature with the shelf life of 3 days. After the shelf life expires, it can be re-filtered once, so that the shelf life will be extended by 3 days after filtration.
(2) Mobile phase B: 100% acetonitrile.
(3) Preparation of solutions of reference sample/ sample to be tested: the reference sample/ sample to be tested was diluted in water. The diluted solution of sample to be tested was transferred into a sample vial equipped with an insert. The injection volume was 50 µg.
(4) Preparation of blank control solution: an appropriate amount of water as a blank control solution was transferred into a sample vial equipped with an insert.
(5) 10% pump washing solution: 100 mL of methanol was taken and added with water to make up to 1 L. The solution was stored at room temperature with the shelf life of 1 month.
(6) Needle washing solution: water, tightly sealed, stored at room temperature.

b. Experimental method

(1) washing the lines: mobile phases A and B was used in equal proportions for perfusion at a flow rate of 5.0 mL/min for no less than 5 min.
(2) Instrument preparation: The channels was filed with at least 25 mL per channel; the chromatographic column was equilibrated with mobile phase A : B = 90 : 10 at 0.8 mL/min to the baseline level. The equilibration time should be no less than 2 h.
(3) Instrument parameter settings, see Table 3:

Table 3 SEC instrument parameter settings

| Instrument parameters | | Elution gradient | | |
|---|---|---|---|---|
| Injection volume | 50µg | Time (min) | %A | %B |
| Flow rate | 0.8mL/min | 0 | 90 | 10 |
| Sample temperature | 10°C | 18 | 90 | 10 |
| Detection wavelength | 280nm | / | / | / |
| Detection time | 18min | / | / | / |
| Column temperature | 25°C | / | / | / |

(4) The injection order is shown in Table 4:

Table 4 Injection order of SEC-HPLC method

| Order | Sample | Injection times | Remark |
|---|---|---|---|
| 1 | Reference solution | 5 | System suitability sample |
| 2 | Sample solution | 1 | Total injection times ≤ 20 |
| 3 | Reference solution | 1 | System suitability sample |

Note: If there are more than 20 samples, repeat Order 2 and 3.

(5) washing and storage of the chromatographic column: Afterwashing with the mobile phase at a flow rate of 0.8 mL/min for 30 min, the chromatographic column was washed with water at a flow rate of 0.5 mL/min for 60 min and stored in water.

c. Data analysis:

For peak definitions, see Table 5.

Table 5 Peak definitions for SEC-HPLC method

| Abbreviation | Name | Desription |
|---|---|---|
| HMW | High molecular weight substances | Peak group preceding the main peak |
| Main Peak | Main Peak | Peak with the largest peak area |
| LMW | Low molecular weight substances | Peak group following the main peak |

$$\text{Area(\%)} = \frac{\text{Area of each peak (group)}}{\text{Total peak area}} \times 100\%$$

Tm value determination: The sample was diluted in 1×PBS to a concentration of 0.2 mg/ml, with sypro Orange dye added to the diluted sample to obtain a final concentration of the dye of 5×. The sample was tested on an instrument (instrument model: Q3, brand: Thermo Scientific). The results are shown in Table 6.

Table 6 SEC and DSF test results for bispecific antibodies

| Sample name | SEC | | | DSF | Sample name | SEC | | | DSF |
|---|---|---|---|---|---|---|---|---|---|
| | Polymer% | Main peak% | Small molecule fragment% | Tm (°C) | | Polymer% | Main peak% | Small molecule fragment% | Tm (°C) |
| BM#2 | 8.36 | 91.64 | 0 | 64.9 | BM#20 | 3.55 | 94.45 | 2 | 62.4 |
| BM#3 | 5.67 | 93.44 | 0.89 | 62.9 | BM#21 | 2.36 | 96.49 | 1.15 | 61.6 |
| BM#4 | 2.45 | 93.68 | 3.87 | 61.7 | BM#22 | 2.36 | 95.79 | 1.85 | 61.5 |
| BM#5 | 2.81 | 97.19 | 0 | 63.6 | BM#23 | 2.59 | 92.42 | 4.99 | 63.5 |
| BM#6 | 5.67 | 94.33 | 0 | 62.4 | BM#24 | 3.61 | 96.39 | 0 | 62 |
| BM#7 | 2.13 | 95.88 | 1.99 | 62.9 | BM#25 | 1.98 | 97.45 | 0.57 | 62.3 |
| BM#8 | 0 | 100 | 0 | 60.5 | BM#27 | 1.7 | 97.42 | 0.88 | 62 |
| BM#9 | 0 | 99.27 | 0.73 | 63.4 | BM#29 | 2.26 | 96.7 | 1.04 | 61.5 |
| BM#10 | 0 | 100 | 0 | 62.4 | BM#30 | 2.45 | 94.57 | 2.98 | 60 |
| BM#11 | 2.21 | 97.79 | 0 | 63.7 | BM#31 | 2.57 | 96.13 | 1.3 | 62.3 |
| BM#15 | 2.93 | 97.07 | 0 | 62.9 | BM#32 | 3.56 | 95.72 | 0.72 | 63.7 |
| BM#17 | 4.39 | 90.58 | 5.03 | 62.9 | BM#34 | 0 | 100 | 0 | 61.7 |
| BM#18 | 3.17 | 94.4 | 2.43 | 61.8 | BM#35 | 1.71 | 98.29 | 0 | 61.7 |
| BM#19 | 2.25 | 93.52 | 4.23 | 63.6 | BM#37 | 1.38 | 98.62 | 0 | 60.4 |

Example 7: Non-reducing capillary electrophoresis (nCE) to detect the purity of bispecific antibodies

The non-reducing capillary electrophoresis purity detection method was performed using a high voltage electric field as the driving force, and an elastic quartz capillary tube (17cm) filled with gel as the separation channel. The gel will form a molecular sieve in the capillary tube, and the samples having different molecular weights will be separated due to different moving speeds in the capillary. The response value is detected by a UV detector, and its purity is calculated based on the percentage of the corrected peak area of each component.

In the present disclosure, Protein Simple capillary electrophoresis instrument (Maurice) was used for nCE detection of antibody, and preferably bispecific antibodies BM#9, BM#10 and BM#34 with a purity of nearly 100% in SEC detection results. First, the reference sample and samples to be tested were diluted to 2.5±0.1 mg/ml in Maurice 1X Sample Buffer, with a total volume of not less than 40 $\mu$l; 55 $\mu$l Maurice 1X Sample Buffer and 5 ul iodoacetamide solution were added respectively; the above solutions were each mixed, and short-spinned for 5 seconds, and the centrifuge tube was placed in a 70°C water bath for 10 minutes, mixed by shaking, and cooled to room temperature; after centrifugation at 12000 rpm for 60 seconds, 80 $\mu$l of the supernatant was collected to a 96-well plate, and the 96-well plate was placed in a centrifuge at 1000g for 2 min; the 96- well plate was placed in a Maurice metal adapter; the initial conditions of the system were set as follows: sample plate temperature of 15°C, electric injection at 4600v for 20s, separation condition at 5750v for 45min, and the UV detector was used for detection at wavelength 220nm. The results are shown in Figure 8. The abscissa indicates the peak time in minutes, and the ordinate indicates the absorbance. The peak areas are calculated to show that the purities of the bispecific antibodies BM#9, BM#10, and BM#34 are 94.5%, 96.1%, and 98.4%, respectively.

In Figure 8, Figure 8A shows the results of non-reducing capillary electrophoresis detection of the bispecific antibody BM#9; Figure 8B shows the results of non-reducing capillary electrophoresis detection of the bispecific antibody BM#10; and Figure 8C shows the results of non-reducing capillary electrophoresis detection of the bispecific antibody BM#34.

Example 8: FC$\gamma$RIIIa (i.e. Fc$\gamma$IIIa receptor) affinity detection

The binding of the bispecific antibodies BM#1-BM#37 in Example 4 of the present disclosure to the human FC$\gamma$RIIIa receptor was studied using Octet 96 instrument by biomembrane interference technology (BLI). BLI is a technology that detects the surface reaction of the sensor by detecting the displacement change of the interference spectrum. When a beam of visible light is emitted from the spectrometer, two reflection spectra will be formed at the two interfaces of the optical film layer at the end of the sensor, so as to form an interference spectrum. Any changes in the thickness and density of the film layer formed by molecular binding or dissociation can be reflected by the displacement value of the interference spectrum, and through this displacement value, a real-time reaction monitoring spectrum can be made.

The detection steps are as follows:

Preparation of SD buffer: An appropriate amount of Tween 20 was dissolved in 1$\times$ PBS (pH 7.4) so that Tween 20 was in a mass of 0.02%.

(2) Preparation of antibody working solution: 10 $\mu$g/mL antibody (bispecific antibody) in SD buffer was prepared.

(3) Preparation of antigen working solution: 200 nM antigen (FC$\gamma$RIIIa) in SD buffer was first prepared at and then serially diluted in 2 folds to prepare a total of 5 concentration gradients, in addition to which a zero concentration was prepared.

(4) Preparation of glycine stock solution (0.1 M, pH 2.0): 7.5 g of solid glycine was dissolved in an appropriate amount of deionized water, subjected to the pH adjustment to 2.0 with hydrochloric acid, and then make up to 100 mL with deionized water.

(5) Preparation of glycine working solution (0.01 M, pH 2.0): An appropriate amount of glycine stock solution was diluted 10 times with deionized water, and mixed well.

(6) System: Octet 96 was launched as well as the Data Acquisiton software in its supporting computer. The bottom and sides of the acquisition probelens were cleaned using cleaning paper dipped with an appropriate amount of 75% ethanol to clean. The instrument was preheated for more than 15 minutes.

(7) Sensor pre-wetting: Before performing the experiment, the sensor was soaked in SD buffer for more than 10 minutes for later use.

(8) Arrangement of samples to be tested: The samples to be tested was arranged in a 96-well black plate at 200 $\mu$L/well as shown in the table below. This can be adjusted based on actual conditions.

(9) Analysis procedure: As shown in Table 7, the time can be adjusted according to the specific experimental conditions.

Table 7 Analysis procedures

| Steps of the experiment | Time (s) | Sample | Location of Sensor |
|---|---|---|---|
| Baseline1 | 60 | SD buffer | Column 1 |

(continued)

| Steps of the experiment | Time (s) | Sample | Location of Sensor |
|---|---|---|---|
| Loading | 300 | Antibody Sample | 3/4/5/6/7/8/9 |
| Baseline2 | 180 | SD buffer | 2 |
| Association | 60 | Antigen | 10 |
| Dissociation | 400 | SD buffer | 2 |

(10) When the analysis was complete, the instrument and computer were shut down.

(11) Data analysis: related constants, such as KD, Kon, Koff, X2, Full R2 and the like were calculated in Data Analysis software.

In the examples of the present application, the modified bispecific antibody was subjected to BLI test to determine the affinity of the modified bispecific antibody to human FCγRIIIa. The results are shown in Table 8. The detection values for the tested sample were of the same order of magnitude as the control sample hIgG1 (hIgG in Table 8 refers to hIgG1) with non-obvious difference, indicating that the modification of the bispecific antibody of the present disclosure does not affect the binding of the antibody to FCγRIIIa. hIgG1 was purchased commercially. The results of BLI test of the bispecific antibodies are shown in Table 8.

Table 8 BLI test results for bispecific antibodies

| Sample name | BLI | | Sample name | BLI | |
|---|---|---|---|---|---|
| | KD (M) | Ratio | | KD (M) | Ratio |
| BM#1 | 6.89E-08 | 1.29 | BM#20 | 6.39E-08 | 1.39 |
| BM#2 | 7.91E-08 | 1.12 | BM#21 | 8.54E-08 | 1.04 |
| BM#3 | 8.82E-08 | 1.00 | BM#22 | 8.63E-08 | 1.03 |
| BM#4 | 7.42E-08 | 1.19 | BM#23 | 8.21E-08 | 1.08 |
| BM#5 | 7.65E-08 | 1.16 | BM#24 | 8.33E-08 | 1.06 |
| BM#6 | 9.13E-08 | 0.97 | BM#25 | 8.18E-08 | 1.08 |
| BM#7 | 6.10E-08 | 1.45 | BM#26 | 7.69E-08 | 1.15 |
| BM#8 | 6.34E-08 | 1.40 | BM#27 | 9.69E-08 | 0.91 |
| BM#9 | 7.30E-08 | 1.21 | BM#28 | 6.13E-08 | 1.45 |
| BM#10 | 7.09E-08 | 1.25 | BM#29 | 7.39E-08 | 1.20 |
| BM#11 | 4.62E-08 | 1.92 | BM#30 | 6.96E-08 | 1.27 |
| BM#12 | 5.38E-08 | 1.65 | BM#31 | 8.71E-08 | 1.02 |
| BM#13 | 6.25E-08 | 1.42 | BM#32 | 7.60E-08 | 1.17 |
| BM#14 | 5.52E-08 | 1.61 | BM#33 | 7.06E-08 | 1.26 |
| BM#15 | 6.82E-08 | 1.30 | BM#34 | 7.85E-08 | 1.13 |
| BM#16 | 5.94E-08 | 1.49 | BM#35 | 6.49E-08 | 1.36 |
| BM#17 | 6.26E-08 | 1.42 | BM#36 | 6.42E-08 | 1.38 |
| BM#18 | 6.98E-08 | 1.27 | BM#37 | 7.34E-08 | 1.21 |
| BM#19 | 8.08E-08 | 1.10 | hIgG | 8.86E-08 | 1.00 |

Example 9: Expression and purification of tool antibodies targeting CD33 protein (Tool antibody, RT31-Tab4) and CD123 protein (Tool antibody, RT28-Tab4-mut)

The tool antibody RT31-Tab4 targeting CD33 protein has a sequence from WO2015067570A2. The tool antibody RT28-Tab4-mut targeting CD123 protein is Talacotuzumab, which has a sequence from IMGT.

The above sequences were entrusted to General Biosystems (Anhui) Co., Ltd. for codon optimization in mammalian cell expression system and cloned into pcDNA3.1 vector. After screening by resistance, the bacteria with positive plasmid were selected for amplification and extraction of the plasmid using a plasmid midi-prep kit (Macherey Nagel, Cat#740412.50). 100 $\mu$g of plasmid (40 $\mu$g heavy chain + 60 $\mu$g light chain) was added per 100 mL of cells, and transient expression was performed in 293F cells (culture medium: FreeStyle 293 Expression medium, Thermo, Cat#12338026 + F-68, Thermo, Cat#24040032) using PEI; 6 to 24 hours after transfection, 5% volume of 10% Peptone (Sigma, Cat#P0521-100G) was added, and cells was cultured for about 7-8 days at 8% $CO_2$ and 130rpm; when the cell viability dropped to 50%, the expression supernatant was collected and purified using ProteinA (GE, Cat#17-5438-02) gravity column; after dialysis with PBS, the concentration was determined using Nanodrop, the purity was identified by SEC, and the binding ability was verified by indirect ELISA.

The tool antibodies targeting CD33 protein and CD123 protein obtained by this method have a concentration of not less than 2 mg/ml and a purity of greater than 95%.

Example 10: Testing of ADCC function of bispecific antibodies by Reporter method

In the examples of the present application, samples with a purity greater than 95% in CE test results are preferably used for ADCC functional testing of bispecific antibodies.

1. The tumor cells MV-4-11 (peripheral blood B cell leukemia cells) of passage 3-4 after thawing and plated into a 96-well plate at 20,000 cells per well;

2. The target sample to be tested, hIgG (hIgG1 was actually used as a negative control and purchased commercially) and the positive control sample were prepared into a solution with a maximum concentration of 10 $\mu$g/mL. A 10-fold serial dilution was performed to obtain a final concentration series of 0.01 ng/mL, 0.1 ng/mL, 1 ng/mL, 10 ng/mL, 100 ng/mL, 1000 ng/mL, and 10000 ng/mL. The target samples to be tested here included BM#9, BM#10, BM#34, BM#19 and other bispecific antibodies prepared in this application. The positive control sample was the aforementioned tool antibody RT31-Tab4 targeting CD33 protein and the tool antibody RT28-Tab4-mut targeting CD123 protein.

3. The serially diluted antibody solutions were added into the cell culture wells according to the volume of the cell suspension.

4. For the sample wells and E/T wells (antibody concentration of 0), Jurkat-NFAT-luc-Fc$\gamma$RIIIa cells were collected and added into the cell culture wells at 20,000 cells per well.

5. After 6 hours of incubation, cell killing was detected using One-Glo kit and read for luminescence.

6. The fold of induction was calculated as follows: fold of induction= (sample -BG) / (E /T-BG).

7. According to the target cell killing rate and concentration, four-parameter fitting was performed to calculate the EC50 concentration of ADCC mediated by each antibody. The lower the EC50 concentration, the stronger the ADCC function mediated by the bispecific antibody. In the above results, the modified antibodies of the present disclosure all had a certain ADCC function, some of which showed an ADCC enhancement effect, and some antibodies showed an ADCC reduction function.

In Figures 9-10, RT2831 only represents the project number of the bispecific antibody against CD33 and CD123. BM-RT2831-#3 represent the bispecific antibody BM#3; BM-RT2831-#4 represent the bispecific antibody BM#4; BM-RT2831-#5 represent the bispecific antibody BM#5; BM-RT2831-#6 represent the bispecific antibody BM#6; BM-RT2831-#7 represent the bispecific antibody BM#7; BM-RT2831-#8 represent the bispecific antibody BM#8; BM-RT2831-#9 represent the bispecific antibody BM#9; BM-RT2831-#10 represent the bispecific antibody BM#10; BM-RT2831-#15 represent the bispecific antibody BM#15; BM-RT2831-#17 represent the bispecific antibody BM#17; BM-RT2831-#18 represent the bispecific antibody BM#18; BM-RT2831-#19 represent the bispecific antibody BM#19; BM-RT2831-#20 represent the bispecific antibody BM#20; BM-RT2831-#21 represent the bispecific antibody BM#21; BM-RT2831-#22 represent the bispecific antibody BM#22; BM-RT2831-#23 represent the bispecific antibody BM#23; BM-RT2831-#24 represent the bispecific antibody BM#24; BM-RT2831-#25 represent the bispecific antibody BM#25; BM-RT2831-#27 represent the bispecific antibody BM#27; BM-RT2831-#29 represent the bispecific antibody BM#29; BM-RT2831-#30 represent the bispecific antibody BM#30; BM-RT2831-#31 represent the bispecific antibody BM#31; BM-RT2831-#32 represent the bispecific antibody BM#32; BM-RT2831-#34 represent the bispecific antibody BM#34; BM-RT2831-#35 represent the bispecific antibody BM#35; and BM-RT2831-#37 represent the bispecific antibody BM#37. In Figure 9, Figure 9A shows the ADCC functional results of RT31-Tab4, RT28-Tab4-mut, hIgG (i.e., hIgG1), and bispecific antibodies BM#3, BM#4, and BM#5, Figure 9B shows the ADCC functional results of bispecific antibodies BM#6, BM#7, BM#8, BM#9, BM#10, and BM#15, and Figure 9C shows the ADCC functional results of bispecific antibodies BM#17, BM#18, BM#19, BM#20, BM#21, and BM#22.

In Figure 10, Figure 10A shows the ADCC functional results of bispecific antibodies BM#23, BM#24, BM#25, BM#27, BM#29, and BM#30, and Figure 10B shows the ADCC functional results of bispecific antibodies BM # 31, BM#32, BM#34, BM#35, and BM#37.

Although the embodiments of the present disclosure have been disclosed as above, they are not limited to the applications listed in the specification and the implementation modes. They can be fully applied to various fields suitable for the present disclosure. For those familiar with the art, additional modifications can be easily implemented. Therefore, without departing from the general concept defined by the claims and the scope of equivalents, the present disclosure is not limited to specific details.

**Claims**

1. An IgG Fc region, comprising a first variant Fc region polypeptide and a second variant Fc region polypeptide, wherein
   a) the first variant Fc region polypeptide and the second variant Fc region polypeptide are both derived from human IgG Fc region polypeptides, and
   b) the first variant Fc region polypeptide differs from the second variant Fc region polypeptide in amino acid(s); and c) the first variant Fc region polypeptide differs from the human IgG Fc region polypeptide in at least one amino acid, and
   d) the second variant Fc region polypeptide differs from the human IgG Fc region polypeptide in at least one amino acid; the first variant Fc region polypeptide is paired with the second variant Fc region polypeptide.

2. The IgG Fc region of claim 1, wherein

   a) the first variant Fc region polypeptide differs from the human IgG Fc region polypeptide in amino acid(s) located in a CH3 domain of the human IgG Fc region; and
   b) the second variant Fc region polypeptide differs from the human IgG Fc region polypeptide in amino acid(s) located in a CH3 domain of the human IgG Fc region.

3. The IgG Fc region of claim 1 or 2, wherein the human IgG Fc region polypeptide is derived from the Fc region polypeptide of human IgG1, IgG2, IgG3, or IgG4.

4. An antibody comprising an IgG Fc region variant, wherein the antibody is a bispecific or multispecific antibody, and the antibody comprises a first full-length antibody comprising a first antigen-binding region and a modified first variant Fc region polypeptide and a second full-length antibody comprising a second antigen-binding region and a modified second variant Fc region polypeptide, the modified first variant Fc region polypeptide and the modified second variant Fc region polypeptide are each derived from human IgG Fc region polypeptides, and the first antigen-binding region and the second antigen-binding region are configured to recognize different antigens; wherein a carboxyl terminus of the first antigen-binding region is coupled to an amino terminus of the modified first variant Fc region polypeptide, and a carboxyl terminus of the second antigen-binding region is coupled to an amino terminus of the modified second variant Fc region polypeptide; the modified first variant Fc region polypeptide is paired with the modified second variant Fc region polypeptide in a manner selected from the group consisting of:

   1) the modified first variant Fc region polypeptide has an amino acid sequence as set forth in SEQ ID NO: 1, and the modified second variant Fc region polypeptide has an amino acid sequence as set forth in SEQ ID NO: 38; or
   2) the modified first variant Fc region polypeptide has an amino acid sequence as set forth in SEQ ID NO: 2, and the modified second variant Fc region polypeptide has an amino acid sequence as set forth in SEQ ID NO: 39; or
   3) the modified first variant Fc region polypeptide has an amino acid sequence as set forth in SEQ ID NO: 3, and the modified second variant Fc region polypeptide has an amino acid sequence as set forth in SEQ ID NO: 40; or
   4) the modified first variant Fc region polypeptide has an amino acid sequence as set forth in SEQ ID NO: 4, and the modified second variant Fc region polypeptide has an amino acid sequence as set forth in SEQ ID NO: 41; or
   5) the modified first variant Fc region polypeptide has an amino acid sequence as set forth in SEQ ID NO: 5, and the modified second variant Fc region polypeptide has an amino acid sequence as set forth in SEQ ID NO: 42; or
   6) the modified first variant Fc region polypeptide has an amino acid sequence as set forth in SEQ ID NO: 6, and the modified second variant Fc region polypeptide has an amino acid sequence as set forth in SEQ ID NO: 43; or
   7) the modified first variant Fc region polypeptide has an amino acid sequence as set forth in SEQ ID NO: 7, and the modified second variant Fc region polypeptide has an amino acid sequence as set forth in SEQ ID NO: 44; or
   8) the modified first variant Fc region polypeptide has an amino acid sequence as set forth in SEQ ID NO: 8, and the modified second variant Fc region polypeptide has an amino acid sequence as set forth in SEQ ID NO: 45; or
   9) the modified first variant Fc region polypeptide has an amino acid sequence as set forth in SEQ ID NO: 9, and the modified second variant Fc region polypeptide has an amino acid sequence as set forth in SEQ ID NO: 46; or
   10) the modified first variant Fc region polypeptide has an amino acid sequence as set forth in SEQ ID NO: 10, and the modified second variant Fc region polypeptide has an amino acid sequence as set forth in SEQ ID NO: 47; or
   11) the modified first variant Fc region polypeptide has an amino acid sequence as set forth in SEQ ID NO: 11, and the modified second variant Fc region polypeptide has an amino acid sequence as set forth in SEQ ID NO: 48; or

12) the modified first variant Fc region polypeptide has an amino acid sequence as set forth in SEQ ID NO: 12, and the modified second variant Fc region polypeptide has an amino acid sequence as set forth in SEQ ID NO: 49; or

13) the modified first variant Fc region polypeptide has an amino acid sequence as set forth in SEQ ID NO: 13, and the modified second variant Fc region polypeptide has an amino acid sequence as set forth in SEQ ID NO: 50; or

14) the modified first variant Fc region polypeptide has an amino acid sequence as set forth in SEQ ID NO: 14, and the modified second variant Fc region polypeptide has an amino acid sequence as set forth in SEQ ID NO: 51; or

15) the modified first variant Fc region polypeptide has an amino acid sequence as set forth in SEQ ID NO: 15, and the modified second variant Fc region polypeptide has an amino acid sequence as set forth in SEQ ID NO: 52; or

16) the modified first variant Fc region polypeptide has an amino acid sequence as set forth in SEQ ID NO: 16, and the modified second variant Fc region polypeptide has an amino acid sequence as set forth in SEQ ID NO: 53; or

17) the modified first variant Fc region polypeptide has an amino acid sequence as set forth in SEQ ID NO: 17, and the modified second variant Fc region polypeptide has an amino acid sequence as set forth in SEQ ID NO: 54; or

18) the modified first variant Fc region polypeptide has an amino acid sequence as set forth in SEQ ID NO: 18, and the modified second variant Fc region polypeptide has an amino acid sequence as set forth in SEQ ID NO: 55; or

19) the modified first variant Fc region polypeptide has an amino acid sequence as set forth in SEQ ID NO: 19, and the modified second variant Fc region polypeptide has an amino acid sequence as set forth in SEQ ID NO: 56; or

20) the modified first variant Fc region polypeptide has an amino acid sequence as set forth in SEQ ID NO: 20, and the modified second variant Fc region polypeptide has an amino acid sequence as set forth in SEQ ID NO: 57; or

21) the modified first variant Fc region polypeptide has an amino acid sequence as set forth in SEQ ID NO: 21, and the modified second variant Fc region polypeptide has an amino acid sequence as set forth in SEQ ID NO: 58; or

22) the modified first variant Fc region polypeptide has an amino acid sequence as set forth in SEQ ID NO: 22, and the modified second variant Fc region polypeptide has an amino acid sequence as set forth in SEQ ID NO: 59; or

23) the modified first variant Fc region polypeptide has an amino acid sequence as set forth in SEQ ID NO: 23, and the modified second variant Fc region polypeptide has an amino acid sequence as set forth in SEQ ID NO: 60; or

24) the modified first variant Fc region polypeptide has an amino acid sequence as set forth in SEQ ID NO: 24, and the modified second variant Fc region polypeptide has an amino acid sequence as set forth in SEQ ID NO: 61; or

25) the modified first variant Fc region polypeptide has an amino acid sequence as set forth in SEQ ID NO: 25, and the modified second variant Fc region polypeptide has an amino acid sequence as set forth in SEQ ID NO: 62; or

26) the modified first variant Fc region polypeptide has an amino acid sequence as set forth in SEQ ID NO: 26, and the modified second variant Fc region polypeptide has an amino acid sequence as set forth in SEQ ID NO: 63; or

27) the modified first variant Fc region polypeptide has an amino acid sequence as set forth in SEQ ID NO: 27, and the modified second variant Fc region polypeptide has an amino acid sequence as set forth in SEQ ID NO: 64; or

28) the modified first variant Fc region polypeptide has an amino acid sequence as set forth in SEQ ID NO: 28, and the modified second variant Fc region polypeptide has an amino acid sequence as set forth in SEQ ID NO: 65; or

29) the modified first variant Fc region polypeptide has an amino acid sequence as set forth in SEQ ID NO: 29, and the modified second variant Fc region polypeptide has an amino acid sequence as set forth in SEQ ID NO: 66; or

30) the modified first variant Fc region polypeptide has an amino acid sequence as set forth in SEQ ID NO: 30, and the modified second variant Fc region polypeptide has an amino acid sequence as set forth in SEQ ID NO: 67; or

31) the modified first variant Fc region polypeptide has an amino acid sequence as set forth in SEQ ID NO: 31, and the modified second variant Fc region polypeptide has an amino acid sequence as set forth in SEQ ID NO: 68; or

32) the modified first variant Fc region polypeptide has an amino acid sequence as set forth in SEQ ID NO: 32, and the modified second variant Fc region polypeptide has an amino acid sequence as set forth in SEQ ID NO: 69; or

33) the modified first variant Fc region polypeptide has an amino acid sequence as set forth in SEQ ID NO: 33, and the modified second variant Fc region polypeptide has an amino acid sequence as set forth in SEQ ID NO: 70; or

34) the modified first variant Fc region polypeptide has an amino acid sequence as set forth in SEQ ID NO: 34, and the modified second variant Fc region polypeptide has an amino acid sequence as set forth in SEQ ID NO: 71; or

35) the modified first variant Fc region polypeptide has an amino acid sequence as set forth in SEQ ID NO: 35, and the modified second variant Fc region polypeptide has an amino acid sequence as set forth in SEQ ID NO: 72; or

36) the modified first variant Fc region polypeptide has an amino acid sequence as set forth in SEQ ID NO: 36, and the modified second variant Fc region polypeptide has an amino acid sequence as set forth in SEQ ID NO: 73; or

37) the modified first variant Fc region polypeptide has an amino acid sequence as set forth in SEQ ID NO: 37, and the modified second variant Fc region polypeptide has an amino acid sequence as set forth in SEQ ID NO: 74.

5. The antibody comprising an IgG Fc region variant of claim 4, wherein the antibody is a bispecific antibody, the first antigen binding region is a first VHH, the second antigen binding region is a second VHH, the first VHH is fused to a modified first variant Fc region polypeptide, and the second VHH is fused to a modified second variant Fc region polypeptide.

6. The antibody comprising an IgG Fc region variant of claim 5, wherein the first antigen binding region targetsCD33 molecule, and the second antigen binding region targets CD123 molecule.

7. The antibody comprising an IgG Fc region variant of claim 6, wherein the first antigen binding region has an amino acid sequence as set forth in SEQ ID NO: 149, and the second antigen binding region has an amino acid sequence as set forth in SEQ ID NO: 150.

8. The antibody comprising an IgG Fc region variant of claim 7, wherein a nucleotide sequence encoding the first antigen binding region is as set forth in SEQ ID NO: 151, and a nucleotide sequence encoding the second antigen binding region is as set forth in SEQ ID NO: 152.

9. A nucleotide molecule encoding an antibody comprising an IgG Fc region variant of any one of claims 4 to 8.

10. The nucleotide molecule of claim 9, wherein the nucleotide sequences encoding the amino acid sequences SEQ ID NOs: 1 to 74 are SEQ ID NOs: 75 to 148, respectively.

11. A vector comprising a nucleotide sequence encoding an antibody comprising an IgG Fc region variant according to any one of claims 4 to 8, or a nucleotide molecule of claim 9 or 10.

12. A host cell, which is capable of expressing an antibody comprising an IgG Fc region variant according to any one of claims 4 to 8, or comprises a vector of claim 11.

13. The host cell of claim 12, wherein the host cell comprises a prokaryotic cell, a eukaryotic cell, or a bacteriophage.

14. A method for producing an antibody comprising: (a) culturing a host cell of claim 12 and (b) recovering the antibody.

15. An immunoconjugate comprising an antibody comprising an IgG Fc region variant according to any one of claims 4 to 8.

16. The immunoconjugate of claim 15 comprising a cytotoxin, a biologically active protein, or a radioactive isotope.

17. A pharmaceutical composition comprising an antibody comprising an IgG Fc region variant according to any one of claims 4 to 8 and a pharmaceutically acceptable carrier.

18. An agent for treating cancer comprising an antibody comprising an IgG Fc region variant according to any one of claims 4 to 8 as an active ingredient.

19. Use of an antibody comprising an IgG Fc region variant according to any one of claims 4 to 8 or a pharmaceutical composition of claim 18 in the preparation of a medicament for treating a disease.

20. The use of claim 19, wherein the disease is a cancer.

21. The use of claim 20, wherein the disease is acute myeloid leukemia.

| 216 | 217 | 218 | 219 | 220 | 221 | 222 | 223 | 224 | 225 | 226 | 227 | 228 | 229 | 230 | 231 | 232 | 233 | 234 | 235 | 236 | 237 | 238 | 239 | 240 | 241 |
|---|---|---|---|---|---|---|---|---|---|---|---|---|---|---|---|---|---|---|---|---|---|---|---|---|---|
| E | P | K | S | C | D | K | T | H | T | C | P | P | C | P | A | P | E | L | L | G | G | P | S | V | F |
| 242 | 243 | 244 | 245 | 246 | 247 | 248 | 249 | 250 | 251 | 252 | 253 | 254 | 255 | 256 | 257 | 258 | 259 | 260 | 261 | 262 | 263 | 264 | 265 | 266 | 267 |
| L | F | P | P | K | P | K | D | T | L | M | I | S | R | T | P | E | V | T | C | V | V | V | D | V | S |
| 268 | 269 | 270 | 271 | 272 | 273 | 274 | 275 | 276 | 277 | 278 | 279 | 280 | 281 | 282 | 283 | 284 | 285 | 286 | 287 | 288 | 289 | 290 | 291 | 292 | 293 |
| H | E | D | P | E | V | K | F | N | W | Y | V | D | G | V | E | V | H | N | A | K | T | K | P | R | E |
| 294 | 295 | 296 | 297 | 298 | 299 | 300 | 301 | 302 | 303 | 304 | 305 | 306 | 307 | 308 | 309 | 310 | 311 | 312 | 313 | 314 | 315 | 316 | 317 | 318 | 319 |
| E | Q | Y | N | S | T | Y | R | V | V | S | V | L | T | V | L | H | Q | D | W | L | N | G | K | E | Y |
| 320 | 321 | 322 | 323 | 324 | 325 | 326 | 327 | 328 | 329 | 330 | 331 | 332 | 333 | 334 | 335 | 336 | 337 | 338 | 339 | 340 | 341 | 342 | 343 | 344 | 345 |
| K | C | K | V | S | N | K | A | L | P | A | P | I | E | K | T | I | S | K | A | K | G | Q | P | R | E |
| 346 | 347 | 348 | 349 | 350 | 351 | 352 | 353 | 354 | 355 | 356 | 357 | 358 | 359 | 360 | 361 | 362 | 363 | 364 | 365 | 366 | 367 | 368 | 369 | 370 | 371 |
| P | Q | V | Y | T | L | P | P | S | R | D | E | L | T | K | N | Q | V | S | L | T | C | L | V | K | G |
| 372 | 373 | 374 | 375 | 376 | 377 | 378 | 379 | 380 | 381 | 382 | 383 | 384 | 385 | 386 | 387 | 388 | 389 | 390 | 391 | 392 | 393 | 394 | 395 | 396 | 397 |
| F | Y | P | S | D | I | A | V | E | W | E | S | N | G | Q | P | E | N | N | Y | K | T | T | P | P | V |
| 398 | 399 | 400 | 401 | 402 | 403 | 404 | 405 | 406 | 407 | 408 | 409 | 410 | 411 | 412 | 413 | 414 | 415 | 416 | 417 | 418 | 419 | 420 | 421 | 422 | 423 |
| L | D | S | D | G | S | F | F | L | Y | S | K | L | T | V | D | K | S | R | W | Q | Q | G | N | V | F |
| 424 | 425 | 426 | 427 | 428 | 429 | 430 | 431 | 432 | 433 | 434 | 435 | 436 | 437 | 438 | 439 | 440 | 441 | 442 | 443 | 444 | 445 | 446 | 447 | 448 | |
| S | C | S | V | M | H | E | A | L | H | N | H | Y | T | Q | K | S | L | S | L | S | P | G | K | – | |

Figure 1

Figure 2

Figure 3

Figure 4

Figure 5

Figure 6

Figure 7

Figure 8

9A

RT28-31 dVHH-hFc mediated ADCC on MV-4-11
ADCC reporter assay
20211229

|  | RT28-Tab4-mut | RT31-Tab4 | hIgG | BM-RT2831-#3 | BM-RT2831-#4 | BM-RT2831-#5 |
|---|---|---|---|---|---|---|
| EC50 | 9.833 | ~ 110310 | ~ 0.000 | 12.00 | ~ 117884293 | ~ 4930631 |

9B

RT28-31 dVHH-hFc mediated ADCC on MV-4-11
ADCC reporter assay
20211229

|  | BM-RT2831-#6 | BM-RT2831-#7 | BM-RT2831-#8 | BM-RT2831-#9 | BM-RT2831-#10 | BM-RT2831-#15 |
|---|---|---|---|---|---|---|
| EC50 | 23.71 | 18.92 | 30.00 | 22.13 | 24.91 | 17.63 |

9C

RT28-31 dVHH-hFc mediated ADCC on MV-4-11
ADCC reporter assay
20211229

|  | BM-RT2831-#17 | BM-RT2831-#18 | BM-RT2831-#19 | BM-RT2831-#20 | BM-RT2831-#21 | BM-RT2831-#22 |
|---|---|---|---|---|---|---|
| EC50 | ~ 682162854 | 13.22 | 14.20 | 14.54 | 27.85 | 44.67 |

Figure 9

10A

**RT28-31 dVHH-hFc mediated ADCC on MV-4-11**
**ADCC reporter assay**
**20211229**

|      | BM-RT2831-#23   | BM-RT2831-#24 | BM-RT2831-#25 | BM-RT2831-#27 | BM-RT2831-#29 | BM-RT2831-#30 |
|------|-----------------|---------------|---------------|---------------|---------------|---------------|
| EC50 | ~ 1326737365    | 53.65         | 58.40         | 13.25         | 1275          | ~ 9360169     |

10B

**RT28-31 dVHH-hFc mediated ADCC on MV-4-11**
**ADCC reporter assay**
**20211229**

|      | BM-RT2831-#31 | BM-RT2831-#32 | BM-RT2831-#34 | BM-RT2831-#35 | BM-RT2831-#37 |
|------|---------------|---------------|---------------|---------------|---------------|
| EC50 | 24.09         | 22.74         | ~ 13.06       | 160.6         | 21.08         |

Figure 10

## INTERNATIONAL SEARCH REPORT

| International application No. |
| --- |
| **PCT/CN2022/117681** |

### A. CLASSIFICATION OF SUBJECT MATTER

C07K 16/46(2006.01)i; C12N 15/13(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

CO7K; C12N; A61K; A61P

Documentation searched other than minimum documentation to the extent that such documents are included in the fields searched

Electronic data base consulted during the international search (name of data base and, where practicable, search terms used)

Data base: CNABS, SIPOABS, DWPI, CNTXT, WOTXT, USTXT, EPTXT, EPTXTC, CNKI, PUBMED, ISI web of science, Genebank, EMBL, STN, 万方数据库, WANFANG DATABASE, 百度学术, BAIDU SCHOLAR, 中国专利生物序列检索系统, China Patent Biological Sequence Search System. Search terms: 双特异抗体, 多特异抗体, 二价抗体, 多价抗体, 双功能抗体, 多功能抗体, 双抗 , 双特异性抗体, 多特异性抗体, 异二聚体抗体, 异源二聚体抗体, 异源二聚化, 单域抗体, 驼源抗体, 骆驼抗体, bispecific, antibod+, bsFv, double specificity, Heterodimeric, bispecific affinity reagent, T366W, D399V, F405T, T366S, L368A, Y407V, K409W, Fc, VHH, CD33, CD123; 对SEQ ID No: 1, 38, 149-152进行序列检索, search for SEQ ID No: 1, 38, 149-152.

### C. DOCUMENTS CONSIDERED TO BE RELEVANT

| Category* | Citation of document, with indication, where appropriate, of the relevant passages | Relevant to claim No. |
| --- | --- | --- |
| X | US 2013336973 A1 (ZYMEWORKS INC.) 19 December 2013 (2013-12-19) see description paragraphs 149, 156-194, 236-239, tables 1-7, figures 1-13. | 1-3 |
| X | CN 110573530 A (ADIMAB LLC) 13 December 2019 (2019-12-13) see description paragraphs 15, 128-147 | 4-5 (all in part), 9-21 (all in part) and 1-3 |
| Y | US 2010291112 A1 (KELLNER, C. et al.) 18 November 2010 (2010-11-18) see claims 1-33 | 6 (in part) |
| X | CN 104955953 A (AJOU UNIVERSITY INDUSTRY COOPERATION FOUNDATION) 30 September 2015 (2015-09-30) see description, embodiment 3 | 4-5 (all in part), 9-21 (all in part) and 1-3 |
| X | US 2018346600 A1 (AJOU UNIVERSITY INDUSTRY-ACADEMIC COOPERATION FOUNDATION) 06 December 2018 (2018-12-06) see description, embodiment 2 | 4-5 (all in part), 9-21 (all in part) and 1-3 |

☑ Further documents are listed in the continuation of Box C.    ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **13 December 2022** | **19 January 2023** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/117681** |

### C.    DOCUMENTS CONSIDERED TO BE RELEVANT

| Category* | Citation of document, with indication, where appropriate, of the relevant passages | Relevant to claim No. |
| --- | --- | --- |
| A | US 2019359710 A1 (FRIEDRICH-ALEXANDER-UNIVERSITÄT ERLANGEN-NÜRNBERG) 28 November 2019 (2019-11-28)<br>        see claims 1-15 | 4-21 (in part), 1-3 |
| X | CN 110944651 A (ADIMAB LLC) 31 March 2020 (2020-03-31)<br>        see description, paragraphs 142-162 | 4-5 (all in part), 9-21 (all in part) and 1-3 |
| Y | CN 110573530 A (ADIMAB LLC) 13 December 2019 (2019-12-13)<br>        see description paragraphs 15, 128-147 | 6 (in part) |
| A | GUY, D.G. et al. "Bispecific Antibodies for the Treatment of Acute Myeloid Leukemia"<br>*Curr Hematol Malig Rep,* 31 December 2018 (2018-12-31),<br>        see pages 417-425 | 4-21 (in part), 1-3 |

Form PCT/ISA/210 (second sheet) (January 2015)

**INTERNATIONAL SEARCH REPORT**

International application No.

**PCT/CN2022/117681**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence listing is an XML file in Standard ST.26.

Form PCT/ISA/210 (continuation of first sheet) (January 2015)

## INTERNATIONAL SEARCH REPORT

International application No.

**PCT/CN2022/117681**

**Box No. III    Observations where unity of invention is lacking (Continuation of item 3 of first sheet)**

This International Searching Authority found multiple inventions in this international application, as follows:

[1]  Invention 1: claims 4-21 relate to relevant technical solutions of an amino acid sequence of a modified first variant Fc region polypeptide being SEQ ID NO: 1, and an amino acid sequence of a modified second variant Fc region polypeptide being SEQ ID NO: 38.

[2]  Invention 2: claims 4-21 relate to relevant technical solutions of an amino acid sequence of a modified first variant Fc region polypeptide being SEQ ID NO: 2, and an amino acid sequence of a modified second variant Fc region polypeptide being SEQ ID NO: 39.

[3]  ...

[4]  Invention 37: claims 4-21 relate to relevant technical solutions of an amino acid sequence of a modified first variant Fc region polypeptide being SEQ ID NO: 37, and an amino acid sequence of a modified second variant Fc region polypeptide being SEQ ID NO: 74.

[5]  Invention 38: claims 4-21 relate to relevant technical solutions of a first variant Fc region polypeptide and a second variant Fc region polypeptide other than that of inventions 1-37.

[6]  The same or corresponding technical features among the above-mentioned inventions are claims 1-3, and "an antibody comprising an IgG Fc region variant, wherein the antibody is a bispecific or multispecific antibody, the antibody comprises a first full-length antibody and a second full-length antibody, the first full-length antibody comprises a first antigen-binding region and a modified first variant Fc region polypeptide, the second full-length antibody comprises a second antigen-binding region and a modified second variant Fc region polypeptide, the modified first variant Fc region polypeptide and the modified second variant Fc region polypeptide are respectively derived from human IgG Fc region polypeptides, and the first antigen-binding region and the second antigen-binding region are used for recognition of different antigens; a carboxyl terminal of the first antigen-binding region is coupled with an amino terminal of the modified first variant Fc region polypeptide, and a carboxyl terminal of the second antigen-binding region is coupled with an amino terminal of the modified second variant Fc region polypeptide; and the modified first variant Fc region polypeptide is paired with the modified second variant Fc region polypeptide". As described below, claims 1-3 and the features of "an antibody comprising an IgG Fc region variant, wherein the antibody is a bispecific or multispecific antibody, the modified first variant Fc region polypeptide and the modified second variant Fc region polypeptide are respectively derived from human IgG Fc region polypeptides, and the modified first variant Fc region polypeptide is paired with the modified second variant Fc region polypeptide" lack novelty; and the features of "an antibody comprising an IgG Fc region variant, wherein the antibody comprises a first full-length antibody and a second full-length antibody, the first full-length antibody comprises a first antigen-binding region and a modified first variant Fc region polypeptide, the second full-length antibody comprises a second antigen-binding region and a modified second variant Fc region polypeptide, and the first antigen-binding region and the second antigen-binding region are used for recognition of different antigens; and a carboxyl terminal of the first antigen-binding region is coupled with an amino terminal of the modified first variant Fc region polypeptide, and a carboxyl terminal of the second antigen-binding region is coupled with an amino terminal of the modified second variant Fc region polypeptide" do not involve an inventive step. Therefore, the above-mentioned inventions do not have a same or corresponding special technical feature, and thus do not comply with the requirement of unity of invention (PCT Rule 13.1).

[7]  D1: US 2013336973 A1, publication date: 19 December 2013, see description, paragraphs 149, 156-194 and 236-239, tables 1-7, and figures 1-13

[8]  D1 discloses a bispecific heteromultimer, comprising a single-domain antigen-binding construct (equivalent to an VHH) derived from a camelidae heavy chain antibody... An isolated heteromultimer comprising at least one heavy chain variable domain and a heterodimeric Fc region are provided, wherein the heterodimeric Fc region comprises a variant CH3 domain, which comprises amino acid mutations that promote the formation of a heterodimer with increased stability... The Fc region is a human IgG Fc region,... and the human IgG Fc region is a human IgG1, IgG2, IgG3 or IgG4 Fc region... A variant Fc heterodimer comprising an immunoglobulin heavy chain variable region and a variant CH3 domain is a multispecific antibody,... in particular, the antibody of the present invention is a bispecific antibody... With regard to the listing of variant CH3 domain mutations, see table 1, table 6 and table 7. Firstly, D1 discloses the variant Fc heterodimer, wherein the CH3 amino acid mutations of the Fc region are beneficial to the formation of a heterodimer; and the variant Fc heterodimer may be a bispecific antibody or a multispecific antibody. Therefore, claims 1-3, and the features of "an antibody comprising an IgG Fc region variant, wherein the antibody is a bispecific or multispecific antibody,

Form PCT/ISA/210 (continuation of first sheet) (January 2015)

INTERNATIONAL SEARCH REPORT

International application No.

**PCT/CN2022/117681**

| Box No. III | Observations where unity of invention is lacking (Continuation of item 3 of first sheet) |
|---|---|

the modified first variant Fc region polypeptide and the modified second variant Fc region polypeptide are respectively derived from human IgG Fc region polypeptides, and the modified first variant Fc region polypeptide is paired with the modified second variant Fc region polypeptide" lack novelty and do not comply with PCT Article 33(2). Secondly, the features of "an antibody comprising an IgG Fc region variant, wherein the antibody comprises a first full-length antibody and a second full-length antibody, the first full-length antibody comprises a first antigen-binding region and a modified first variant Fc region polypeptide, the second full-length antibody comprises a second antigen-binding region and a modified second variant Fc region polypeptide, and the first antigen-binding region and the second antigen-binding region are used for recognition of different antigens; and a carboxyl terminal of the first antigen-binding region is coupled with an amino terminal of the modified first variant Fc region polypeptide, and a carboxyl terminal of the second antigen-binding region is coupled with an amino terminal of the modified second variant Fc region polypeptide" are conventional technical means in the art. Therefore, the above-mentioned technical solution does not involve an inventive step and does not comply with PCT Article 33(3).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **4-21 (all in part), 1-3**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

INTERNATIONAL SEARCH REPORT
Information on patent family members

International application No.

**PCT/CN2022/117681**

| Patent document cited in search report | | | Publication date (day/month/year) | Patent family member(s) | | | Publication date (day/month/year) |
|---|---|---|---|---|---|---|---|
| US | 2013336973 | A1 | 19 December 2013 | WO | 2013166594 | A1 | 14 November 2013 |
| | | | | JP | 2015522525 | A | 06 August 2015 |
| | | | | CA | 2872540 | A1 | 14 November 2013 |
| | | | | US | 2016257763 | A1 | 08 September 2016 |
| | | | | AU | 2013258834 | A1 | 27 November 2014 |
| | | | | EP | 2847230 | A1 | 18 March 2015 |
| | | | | ES | 2843054 | T3 | 15 July 2021 |
| CN | 110573530 | A | 13 December 2019 | KR | 20190120775 | A | 24 October 2019 |
| | | | | WO | 2018152516 | A1 | 23 August 2018 |
| | | | | AU | 2018220734 | A1 | 12 September 2019 |
| | | | | IL | 268790 | A | 31 October 2019 |
| | | | | JP | 2020510646 | A | 09 April 2020 |
| | | | | SG | 11201907638 Q | A | 27 September 2019 |
| | | | | US | 2021130471 | A1 | 06 May 2021 |
| | | | | RU | 2019129174 | A | 22 March 2021 |
| | | | | CA | 3054078 | A1 | 23 August 2018 |
| | | | | EP | 3583131 | A1 | 25 December 2019 |
| | | | | MA | 47508 | A | 17 March 2021 |
| | | | | BR | 112019017277 | A2 | 14 April 2020 |
| US | 2010291112 | A1 | 18 November 2010 | CA | 2694721 | A1 | 15 January 2009 |
| | | | | CN | 101802010 | A | 11 August 2010 |
| | | | | WO | 2009007124 | A1 | 15 January 2009 |
| | | | | EP | 2014680 | A1 | 14 January 2009 |
| | | | | ES | 2532381 | T3 | 26 March 2015 |
| | | | | EP | 2185595 | A1 | 19 May 2010 |
| | | | | AU | 2008274494 | A1 | 15 January 2009 |
| CN | 104955953 | A | 30 September 2015 | EA | 201591038 | A1 | 30 November 2015 |
| | | | | EP | 2927321 | A1 | 07 October 2015 |
| | | | | CA | 2892623 | A1 | 05 June 2014 |
| | | | | ES | 2861446 | T3 | 06 October 2021 |
| | | | | US | 2022162342 | A1 | 26 May 2022 |
| | | | | WO | 2014084607 | A1 | 05 June 2014 |
| | | | | US | 2018237541 | A1 | 23 August 2018 |
| | | | | AU | 2013352812 | A1 | 16 July 2015 |
| | | | | KR | 20140067944 | A | 05 June 2014 |
| | | | | US | 2015307628 | A1 | 29 October 2015 |
| | | | | EP | 3878964 | A1 | 15 September 2021 |
| | | | | BR | 112015012310 | A2 | 06 February 2018 |
| | | | | IL | 239041 | D0 | 30 July 2015 |
| | | | | JP | 2016506377 | A | 03 March 2016 |
| | | | | DK | 2927321 | T3 | 15 March 2021 |
| US | 2018346600 | A1 | 06 December 2018 | KR | 20170042943 | A | 20 April 2017 |
| | | | | WO | 2017065484 | A1 | 20 April 2017 |
| | | | | EP | 3363818 | A1 | 22 August 2018 |
| US | 2019359710 | A1 | 28 November 2019 | EP | 3548516 | A1 | 09 October 2019 |
| | | | | CA | 3045385 | A1 | 07 June 2018 |
| | | | | WO | 2018100139 | A1 | 07 June 2018 |
| | | | | EP | 3330289 | A1 | 06 June 2018 |
| CN | 110944651 | A | 31 March 2020 | JP | 2022101709 | A | 06 July 2022 |

Form PCT/ISA/210 (patent family annex) (January 2015)

**INTERNATIONAL SEARCH REPORT**
Information on patent family members

| International application No. |
| :--- |
| **PCT/CN2022/117681** |

| Patent document cited in search report | Publication date (day/month/year) | Patent family member(s) | | Publication date (day/month/year) |
| :---: | :---: | :--- | :---: | :---: |
| | | KR 20190118172 | A | 17 October 2019 |
| | | RU 2019128060 | A | 09 March 2021 |
| | | CA 3053010 | A1 | 16 August 2018 |
| | | JP 2020506971 | A | 05 March 2020 |
| | | IL 268554 | A | 26 September 2019 |
| | | SG 11201907299X | A | 27 September 2019 |
| | | AU 2018219887 | A1 | 22 August 2019 |
| | | US 2019359716 | A1 | 28 November 2019 |
| | | MX 2019009468 | A | 20 January 2020 |
| | | BR 112019016315 | A2 | 31 March 2020 |
| | | EP 3579848 | A1 | 18 December 2019 |
| | | WO 2018148445 | A1 | 16 August 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015067570 A2

**Non-patent literature cited in the description**

- **KABAT et al.** Sequence of Proteins of Immunological Interest. U.S. Dept. of Health and Human Services, 1983